# EUROPEAN PATENT APPLICATION

(11) **EP 4 335 446 A1**
(43) Date of publication of application: **13.03.2024**
(21) Application number: 22778529.2
(22) Date of filing: 11.03.2022
(51) Int. Cl.: A61K 35/30, C12N 5/10, A61P 25/08

(54) **DRUG AND METHOD FOR FORMING GABAERGIC NEURONS**

(30) Priority: 31.03.2021 CN 202110349287
(71) Applicant: Jinan University, Tianhe District Guangzhou Guangdong 510632 (CN)
(72) Inventor: CHEN, Gong, Macau Institute Of CNS Regeneration, Jinan University Guangzhou, Guangdong 510632 (CN); ZHENG, Jiajun, Macau Institute Of CNS Regeneration, Jinan University Guangzhou, Guangdong 510632 (CN); YU, Jiandong, Macau Institute Of CNS Regeneration, Jinan University Guangzhou, Guangdong 510632 (CN)
(74) Representative: CH Kilger Anwaltspartnerschaft mbB
(86) International application number: PCT/CN2022/080444
(87) International publication number: WO 2022/206345

(57) **Abstract**

Provided are a drug and method for treating temporal lobe epilepsy (TLE) in a subject, the method comprising: administering a nucleic acid encoding a NeuroD1 polypeptide to glial cells in hippocampus region of the subject, so that the glial cells express NeuroD1 polypeptides and are transformed into GABAergic neurons. Also provided are a drug and method for forming GABAergic neurons in hippocampus region of a living mammalian brain, the method comprising: administering a nucleic acid encoding NeuroD1 polypeptides to glial cells located in hippocampus region, so that the glial cells express NeuroD1 polypeptides and are transformed into GABAergic neurons.

## Description

### TECHNICAL FIELD

The present invention relates to a drug/medicament and method for treating temporal lobe epilepsy (TLE) in a subject, and a drug/medicament and method for forming GABAergic neurons in hippocampus region of a living mammalian brain.

### BACKGROUND TECHNOLOGIES

Temporal lobe epilepsy (TLE) is one of the most common types of epilepsy, characterized by reduction of inhibitory GABAergic interneurons, hippocampal hyperexcitability and spontaneous recurrent seizures (SRS) commonly originating from the hippocampus region¹. About 35% of patients with TLE are resistant to antiepileptic drugs (AED)², and AED therapy rarely alleviates learning and memory impairments and mood abnormalities in TLE patients³. Cell transplantation therapy has been tested in preclinical models of TLE4. Grafting of medial ganglionic eminence (MGE) GABAergic precursor cells derived from the mouse^{5,6} and human induced pluripotent stem cells (hiPSC)^{7,8} have shown promise in reducing the frequency of SRS. Moreover, transplanting GABAergic precursor cells can rescue learning and memory impairment and mood abnormalities suggesting that increasing GABAergic neuron density will be a promising therapeutic approach for TLE.

Recently, *in vivo* direct reprogramming of glial cells into neurons opened a new avenue for replenishing lost neurons⁹⁻¹⁸. We have reported that NeuroD1 can reprogram mouse cortical NG2 cells into GABAergic neurons¹⁶. Combining NeuroD1 and Dlx2 together can convert striatal astrocytes into GABAergic neurons²⁰. ASCL1 has been shown to reprogram midbrain astrocytes into both glutamatergic and GABAergic neurons²¹. NG2 cells can be reprogrammed into GABAergic neurons by Sox2 in the cortex at low efficiency²² but by ASCL1 + LMX1a + NURR1 in the striatum at high efficency^{23,24}.

There is also a need in the art for methods of generating neurons, particularly GABAergic neurons, to treat diseases associated with reduction or damage of GABAergic neurons.

### SUMMARY OF THE INVENTION

The present invention meets the above needs by providing a medicament and a method for treating temporal lobe epilepsy (TLE) in a subject, and a medicament and a method for forming GABAergic neurons in hippocampus region of a living mammalian brain.

In one aspect, the present invention provides a medicament and a method for treating temporal lobe epilepsy (TLE) in a subject, comprising administering a nucleic acid encoding a NeuroD 1 polypeptide to glial cells in hippocampus region of the subject, so that the glial cells express the NeuroD1 polypeptide and are converted into GABAergic neurons.

In another aspect, the present invention provides a medicament and a method for forming GABAergic neurons in hippocampus region of a living mammalian brain, comprising administering a nucleic acid encoding a NeuroD1 polypeptide to glial cells located in the hippocampus region, so that the glial cells express the NeuroD 1 polypeptide and are converted into GABAergic neurons.

In one aspect, the present invention provides use of a nucleic acid encoding a NeuroD 1 polypeptide in the manufacture of a medicament for the treatment of temporal lobe epilepsy (TLE) in a subject, wherein the treatment comprises administering the nucleic acid encoding the NeuroD1 polypeptide to glial cells in hippocampus region of the subject, so that the glial cells express the NeuroD1 polypeptide and are converted into GABAergic neurons.

In another aspect, the present invention provides use of a nucleic acid encoding a NeuroD 1 polypeptide in the manufacture of a medicament for converting glial cells in hippocampus region of a living mammalian brain into GABAergic neurons, wherein the conversion comprises causing the glial cells to express the NeuroD1 polypeptide and to be converted into GABAergic neurons.

### BRIEF DESCRIPTION OF THE DRAWINGS

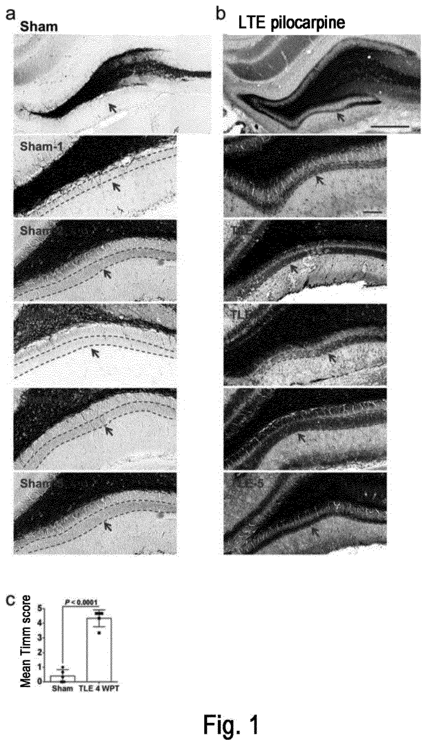

**Fig. 1** **Timm staining of rats in sham group or TLE group.**
   a. Timm staining after saline injection (sham, n = 5 rats). Red line and red arrows showing normal dentate granular layer staining pattern.
   b. Timm staining at 4 weeks after pilocarpine injection (n = 5 rats). Mossy fiber sprouting was seen clearly in all 5 TLE rats (red line and arrows). Scale bar, top 500 µm, below 100 µm.
   c. Mean Timm scores in Sham and TLE groups. Student's t test.

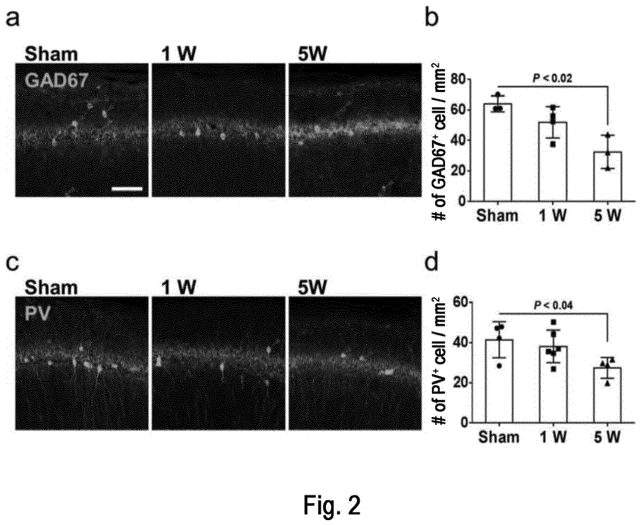

**Fig. 2** **Inhibitory interneurons showed a time-dependent loss in TLE rats.**
   a. GAD67 immunofluorescent images of the CA1 region in rats with saline injection (sham) or pilocarpine injection (1 and 5 weeks). Scale bar, 50 µm.
   b. Quantification of GAD67⁺ cell density in different groups.
   c. PV immunofluorescent images of the CA1 region in rats with saline or pilocarpine injection (1 and 5 weeks).
   d. Quantification of PV⁺ cell density in different groups.
**Fig. 3** **GFAP immunofluorescent images of the hippocampus region in rats without or with TLE.**
   GFAP staining revealed reactive astrocytes at 1 week after pilocarpine injection compared to the sham group (top row). Left column shows enlarged view of the CA1 region (Scale bar, 500 µm). Right column shows the GFAP staining of entire hippocampus region. Scale bar, 50 µm.
**Fig. 4****. Time course of astrocyte-to-neuron conversion induced by NeuroD1 in the hippocampus region of TLE rats.**
   a. Schematic diagram showing experimental design.
   b. AAV9 expression pattern in TLE rat hippocampus region at different time points (1, 3, 6, 9 weeks) after viral injection. Note a clear morphological difference at 6-9 weeks between NeuroD1-GFP and GFP control. Scale bar, 200 µm.
   c. Gradual conversion of astrocytes into neurons revealed by GAFP and NeuN staining from 1 week to 9 weeks after viral injection. Arrowheads indicate NeuroD1-GFP infected cells that are also NeuN⁺. Note that NeuN signal was weak at 1-3 weeks but strong at 6-9 weeks after NeuroD1 infection. Scale bar, 50 µm.
   d, Quantification of NeuroD1-mediated neuronal conversion efficiency, which increased from 1 week to 9 weeks, and 9 WPI GFP control. Each dot represents one rat. Lower panel illustrating representative coronal sections of the hippocampal CA1 region (delineated in red) used in quantitative analyses.

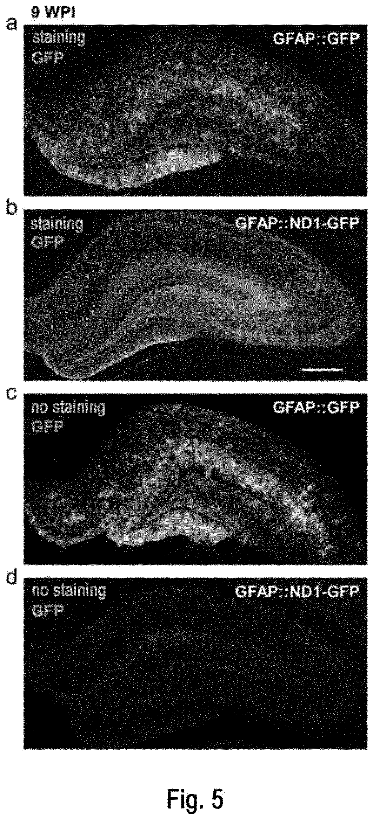

**Fig. 5** **Efficient AAV9 viral infection in the rat hippocampus region.**
   a-b. hippocampus regionInjecting AAV9 GFAP::GFP virus or GFAP::NeuroD1-GFP.
   c-d. GFP fluorescence images without immunostaining after 9 weeks of injecting AAV9 GFAP::GFP virus or GFAP::NeuroD1-GFP. Note the drastic difference of GFP signal, indicating that after NeuroD1-induced astrocyte-to-neuron conversion, the GFAP promoter activity has been largely silenced in hGFAP::NeuroD1-GFP converted neurons. Scale bar, 500 µm.

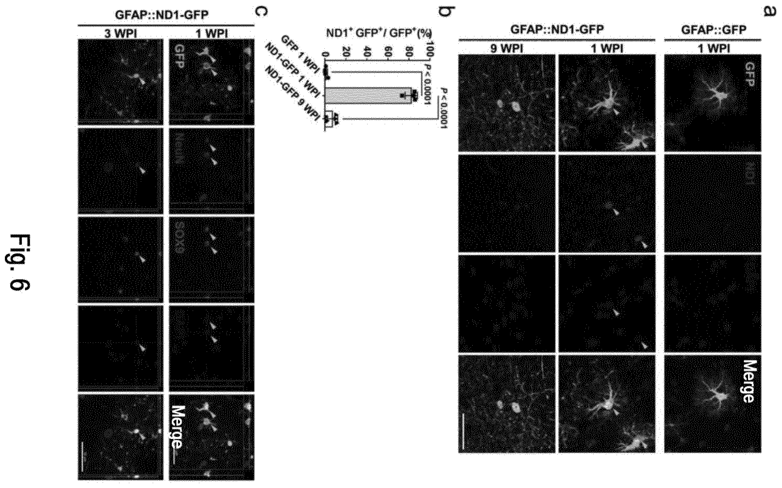

**Fig. 6** **NeuroD1 expression in astrocytes and transitional stage.**
   a. NeuroD1 expression detected in astrocytes at 1 week after injecting AAV9 GFAP::NeuroD1-GFP viruses, but not at 9 weeks when converted neurons have matured. Scale bar, 50 µm.
   b. Quantification of NeuroD1⁺ cells among all GFP⁺ cells.
   c. Confocal images at 1 and 3 weeks after injecting AAV9 GFAP::NeuroD1-GFP viruses. Transitional stage from astrocytes to neurons was revealed by the colocalization of NeuN (red) and SOX9 (magenta) among NeuroD1-GFP infected cells.

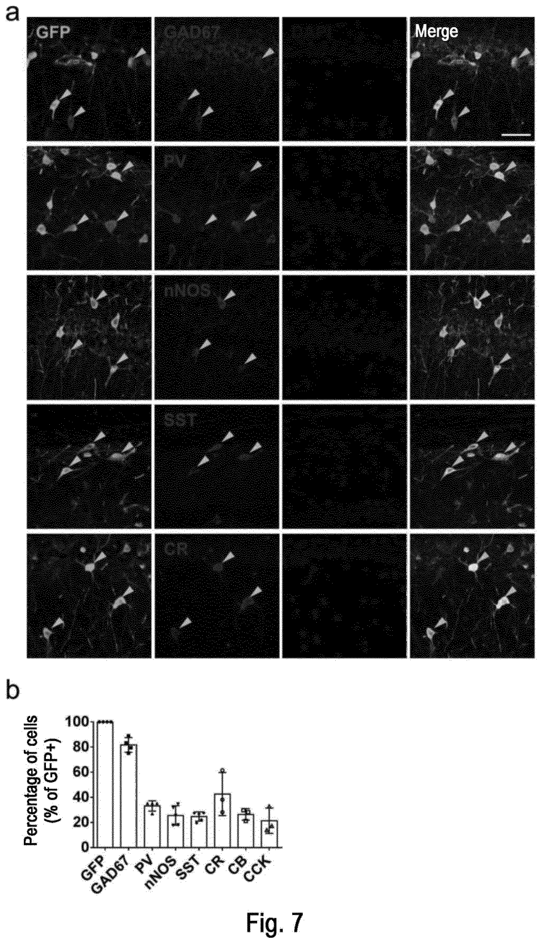

**Fig. 7** **NeuroD1 reprograms hippocampal astrocytes into GABAergic neurons in TLE rats.**
   a. Representative images illustrating NeuroD1-converted cells immunopositive for various GABAergic neuron markers GAD67, PV, nNOS, SST and CR. Scale bar, 50 µm.
   b. Quantified data showing the percentage of each neuronal subtype at 9 weeks after infection. PV: parvalbumin; nNOS: neuronal nitric oxide synthase; SST: somatostatin; CR: calretinin; CB: calbindin; CCK: cholecystokinin. Each dot represents one rat.

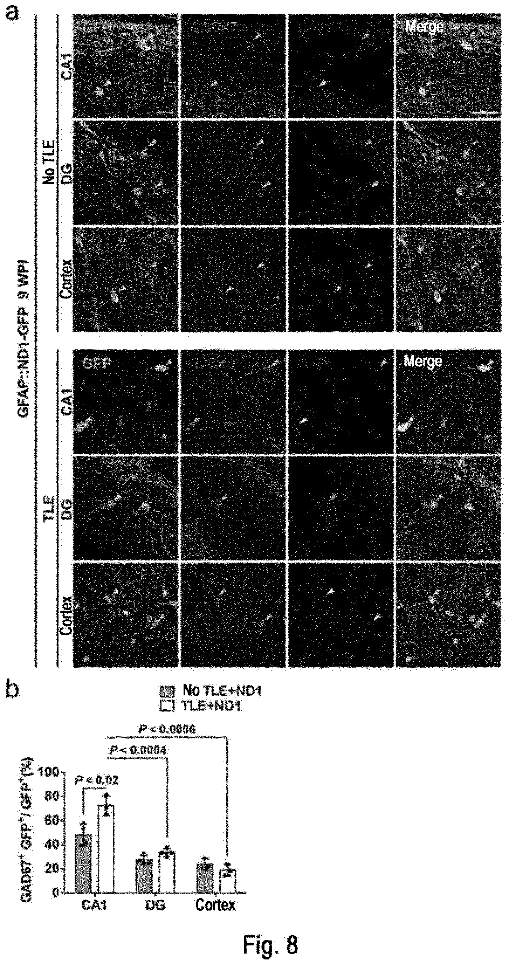

**Fig. 8** **Regional difference of the NeuroD1-converted GABAergic neurons.**
   a. Representative images of GAD67+ neurons and NeuroD1-GFP infected cells in the cortex, hippocampal CA1 and DG areas with or without TLE.
   b. Quantification of the percentage of GAD67⁺ neurons among NeuroD 1-GFP infected cells at 9 WPI in different brain regions. Gray bar, without TLE; white bar, TLE. Note that the percentage of GAD67+ neurons generated by NeuroD 1 is much higher in the CA1 region than that in the DG and cortex. TLE model also showed more GABAergic neurons than that without TLE.

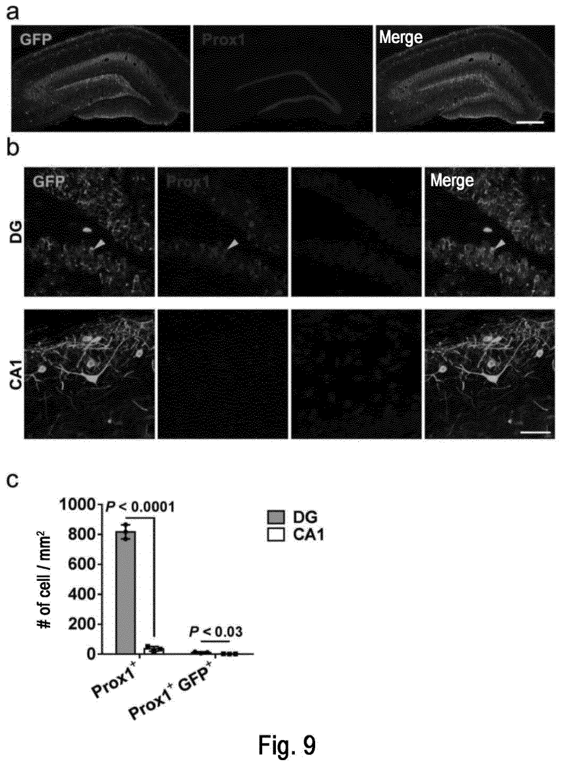

**Fig. 9** **NeuroD1-converted neurons not from adult neural stem cells in the hippocampus region.**
   a. Representative hippocampal section stained with GFP and Prox1 from TLE rats injected with NeuroD1-GFP (9 WPI). Scale bar, 500 µm.
   b. NeuroD1-converted cells show very few Prox1⁺ neurons in the DG and almost none in the CA1 region. Scale bar, 50 µm.
   c. Quantitative data showing very low number of Prox1 positive cells among NeuroD 1-GFP infected cells in the DG, suggesting that NeuroD 1-converted neurons are not from dentate granular progenitor cells (9 WPI in TLE rats, n = 3 rats). Student's *t* test.

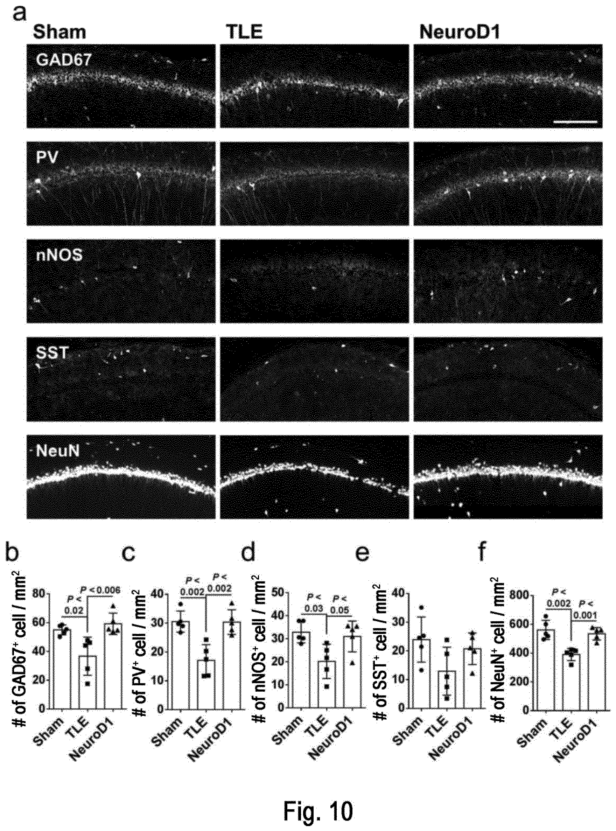

**Fig. 10** **NeuroD1 treatment rescues neuronal loss in TLE rat hippocampus region.**
   a. Representative images illustrating GAD67, PV, nNOS, SST and NeuN staining in the hippocampal CA1 region from sham control, TLE rats, and TLE rats treated with NeuroD1. Scale bar, 200 µm.
   b. Quantification of GAD67+ neuron density (cells per mm²). Note that GABAergic neurons decreased after TLE but were rescued by NeuroD1 treatment.
   c-e. Quantification of PV⁺, nNOS⁺, SST⁺ cells in each group.
   f. Quantification of the total neuronal density in the CA1 region, which also showed a significant reduction after TLE but rescued by NeuroD1. Each dot represents one animal. One way ANOVA followed by Tukey post hoc test.

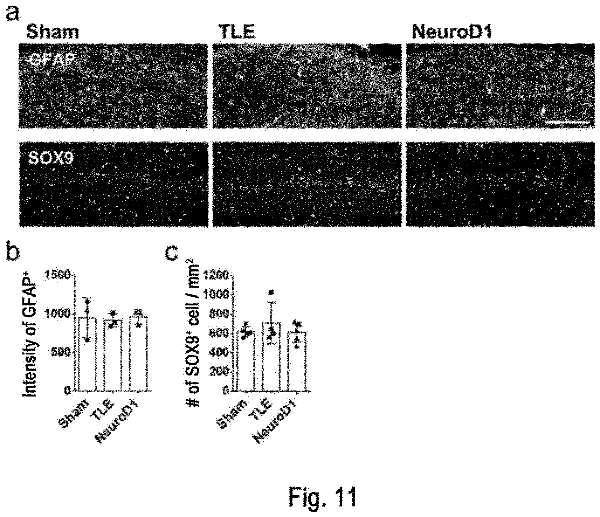

**Fig. 11** **NeuroD1 reprogramming had no effect on the number of astrocytes in epileptic rat hippocampus region.**
   a. Representative GFAP and SOX9 immunostaining images in sham control, TLE rats without or with NeuroD1 treatment at 9 WPI in the CA1 region.
   b-c. Quantification of the intensity of GFAP⁺ cells and the density of SOX9⁺ cells. No significant difference among the 3 groups.

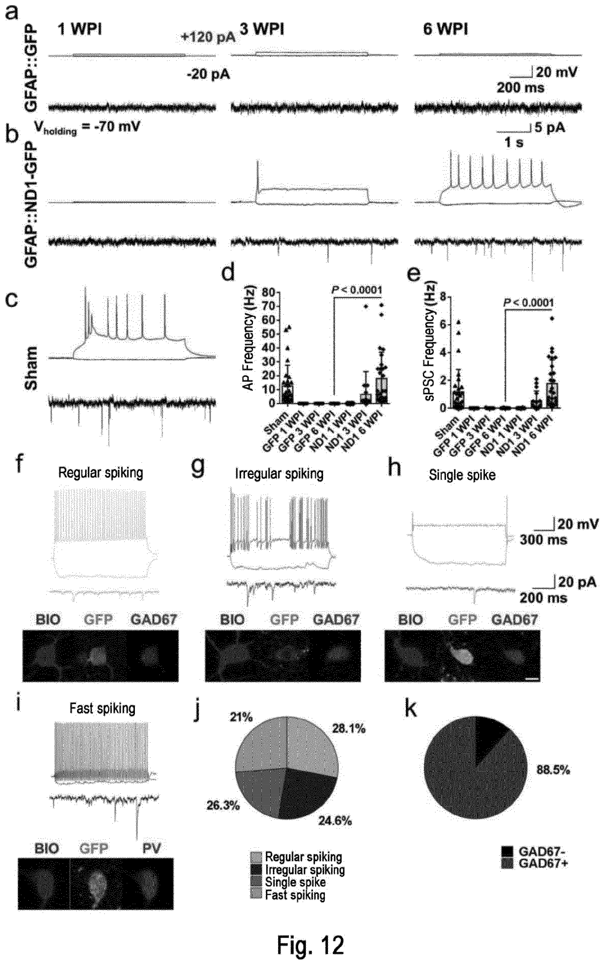

**Fig. 12** **Functional characterization of astrocyte-converted neurons in the TLE rat hippocampus region.**
   a-c. Representative traces illustrating action potential (AP) firing (top row) and spontaneous synaptic events (bottom row) recorded from control GFP-infected cells (a), NeuroD1-GFP infected cells (b), and sham group (c). Action potentials and synaptic events were detected at 3 WPI and increased significantly at 6 WPI, indicating a gradual maturation of newly converted neurons.
   d-e. Bar graphs showing the frequency of action potentials (d) and synaptic events (e) among each group at different time points.
   f-i. Representative traces illustrating different AP firing patterns (top row) and spontaneous synaptic currents (middle row), together with post-recording biocytin immunostaining. The majority of biocytin-labeled cells were GAD67⁺, with a subpopulation being PV⁺ neurons (i, bottom row). Scale bar, 10 µm.
   j. Pie chart showing the proportion of the newly converted neurons displaying four different AP firing patterns (n = 57 cells from 6 rats).
   k. The pie chart showing the percentage of GAD67+ cells among the biocytin-labeled cells (n = 26 cells from 6 rats).

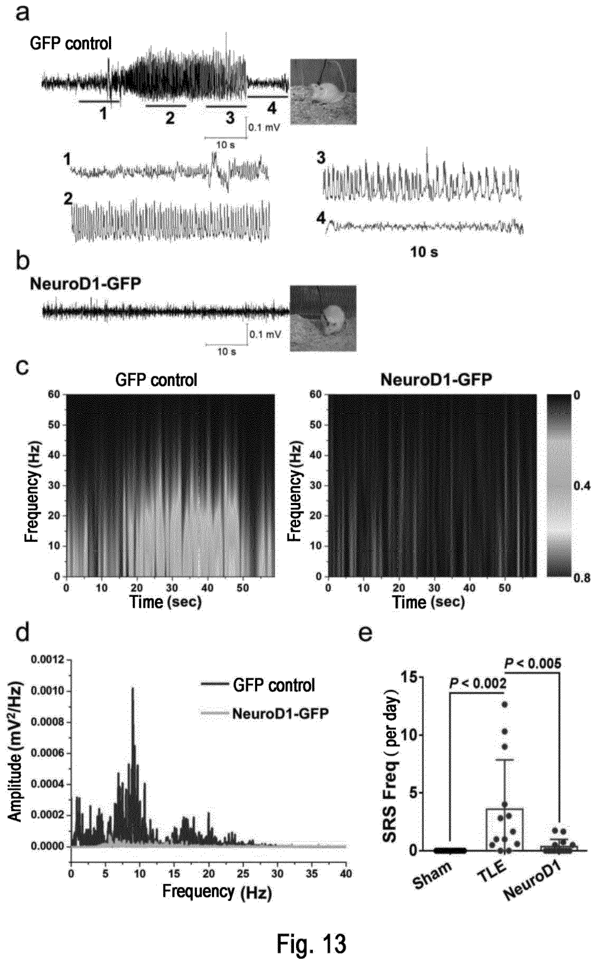

**Fig. 13** **NeuroD1 treatment reduces spontaneous seizures in epileptic rats.**
   a-b. Representative video-EEG recordings in TLE rats injected with GFP control AAV (a) or NeuroD1-GFP AAV (b). Spontaneous seizure activity was frequently observed in the control GFP group but not NeuroD 1-treated rats. Traces 1-4 show the enlarged view of spontaneous seizure activity.
   c. The Short-time Fourier transform of EEG recording data shown in panel a and b (15000 samples with a duration of 60 s). Blue represents low activity, while green and yellow represent high activity.
   d. Power spectrum analysis of the EEG recording data shown in panel a and b.
   e. Quantified data showing the SRS frequency in Sham (n = 13 rats), TLE injected with GFP (n = 13 rats), and TLE injected with NeuroD1 group (n = 14 rats). One way ANOVA followed by Tukey post hoc test.

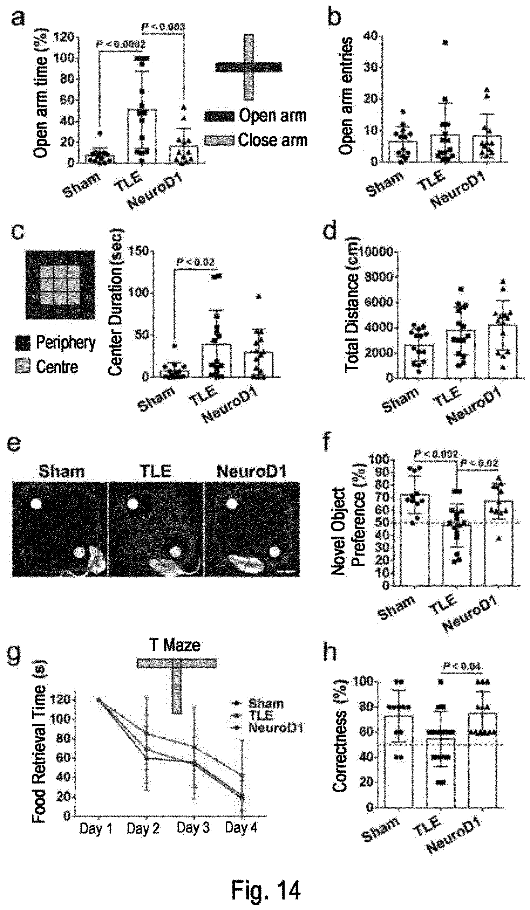

**Fig. 14** **NeuroD1 treatment rescues behavioral abnormalities in epileptic rats.**
   a-b. Elevated plus maze test showing TLE rats spending more time in the open arm than sham group, which was rescued in NeuroD1-treated group (one way ANOVA followed with Tukey post hoc test). The number of entries to the open arm was similar among different groups (b).
   c-d. Open field test showing the mean time spent in the center of the arena increased significantly after induction of TLE (c), with total distance traveled also showed a trend of increase after TLE (d).
   e-f. Novel object recognition test showing TLE rats spending less time near a novel object compared to the sham group, which was partially rescued after NeuroD 1 treatment.
   g-h. T-maze test showing deficits of TLE rats in reaching the reward of food retrieval compared to the sham group, which was rescued by NeuroD1 treatment. One way ANOVA followed by Tukey post hoc test.

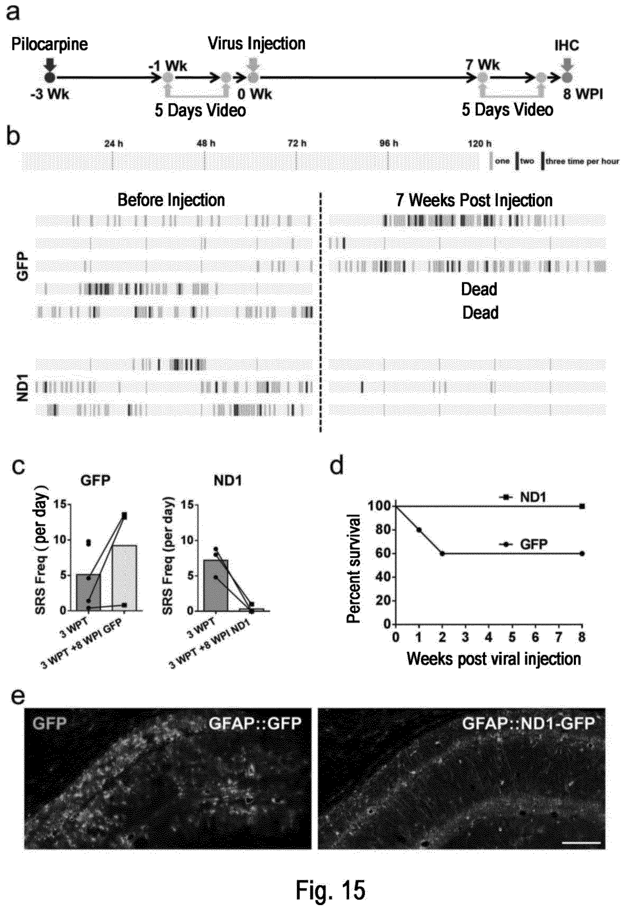

**Fig. 15** **NeuroD1 treatment inhibits spontaneous recurrent seizures after long-term establishment.**
   a. Schematic diagram showing experimental design. AAV was injected at 4 weeks post pilocarpine administration, after spontaneous recurrent seizures being well established in the rat TLE model.
   b. The SRS activity was recorded before and 7 weeks after viral injection (TLE injected with GFP, n = 5 rats; TLE injected with NeuroD1, n = 3 rats).
   c. Quantified data showing significant inhibition of the SRS frequency after NeuroD 1 treatment.
   d. Survival of TLE rats injected with GFP or NeuroD1-GFP AAV.
   e. Typical images of the TLE rat hippocampus region after 8 weeks of infection by AAV9 GFAP::GFP or GFAP::NeuroD1-GFP viruses. Scale bar, 200 µm.

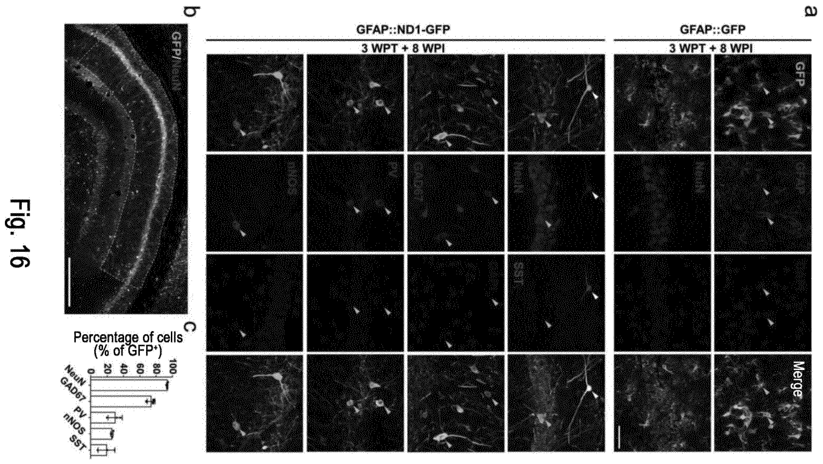

**Fig. 16** **NeuroD1 gene therapy regenerates GABAergic neurons in TLE rats after long-term establishment of SRS.**
   a. Representative immunofluorescent confocal images of viral infected cells (green) co-immunostained with various markers including GFAP, NeuN, GAD67, SST, PV and nNOS. GFP+ cells in control group were colocalized with GFAP. Scale bar, 50 µm.
   b. The quantification area were shown in the IHC image of the rat hippocampal CA1 region.
   c. Quantified data showing the percentage of each neuronal subtype at 8 weeks after NeuroD1-GFP infection. Each dot represents one rat.

### DETAILED DESCRIPTION OF THE INVENTION

The inventors surprisingly found that in the present invention, administration of NeuroD1 alone can convert glial cells (e.g., astrocytes) of hippocampus region into GABAergic neurons at a high rate in the absence or presence of TLE. In the CA1 region of the hippocampus region, the proportion of glial cells converted into GABAergic neurons is particularly high, even reaching about 70%-80%. In the presence of TLE, the conversion effect is better.

Previous studies have shown that NeuroD1 mainly converts astrocytes in the cortex into glutamatergic neurons after ischemic stroke, and the proportion of GABAergic neurons in the converted neurons is about 10%-20%; expression of NeuroD1 polypeptide in striatal astrocytes results in only a smaller number of GABAergic neurons; and the combination of NeuroD 1 and Dlx2 converts striatal astrocytes mainly into GABAergic neurons. To the surprise of the inventors, in the present invention, administration of NeuroD1 alone without combination with other cytokines converts astrocytes in hippocampus region into GABAergic neurons at a high rate in the absence or presence of TLE. In the presence of TLE, the rate of glial cells (such as astrocytes) converted into GABAergic neurons is higher. In the CA1 region of the hippocampus, the rate of glial cells (e.g., astrocytes) converted into GABAergic neurons is particularly high, even reaching about 70%-80%.

In one aspect, in the CA1 region of the hippocampus, the rate of glial cells converted into GABAergic neurons is higher, for example, higher than that in cortical areas (about 10%-20%).

In another aspect, in the CA1 region of the hippocampus, the proportion of glial cells converted into GABAergic neurons is very high, for example, at least about 30% or 40%.

In another aspect, in the CA1 region of the hippocampus, the proportion of glial cells converted into GABAergic neurons is particularly high, for example, at least about 50% or 60%, etc., which is much higher than the proportion of conversion in the cortical area (about 10%-20%).

In another aspect, in the CA1 region of the hippocampus, the proportion of glial cells converted into GABAergic neurons is particularly high, for example, at least about 70%, 80%, 90%, etc., which is much higher than the proportion of conversion in the cortical area (about 10%-20%).

In one aspect, the present invention shows that in the absence or presence of TLE, overexpression of NeuroD1 in glial cells (e.g., astrocytes, e.g., reactive astrocytes) of the hippocampus region (e.g., CA1 region) can directly convert glial cells into GABAergic neurons at high proportions (higher, very high, especially high) as described above. In another aspect, the present invention shows that in the absence or presence of TLE, overexpression of NeuroD1 in glial cells (such as NG2 glial cells, oligodendrocytes, or microglial cells) in the hippocampus region (such as CA1 region) can directly convert glial cells into GABAergic neurons at high proportions as described above. The converted neurons are functional GABAergic neurons, possess both the immunohistological and physiological features of GABAergic interneurons, and are functionally integrated into local synaptic circuits.

In another aspect, NeuroD1-based gene therapy significantly increases GABAergic neuronal density, rescues the loss of neurons in the hippocampus region (such as CA1 region), thereby reducing the frequency of epileptic seizures and inhibiting spontaneous recurrent seizures (SRS). In addition, NeuroD1-based gene therapy can rescue behavioral abnormalities and improve cognitive impairment and mood disorders. The present invention demonstrates that regeneration of GABAergic interneurons through NeuroD1-based gene therapy provides a new approach for the treatment of temporal lobe epilepsy (TLE), such as drug-resistant temporal lobe epilepsy.

Deficits of GABAergic neurons are one of the major causes of TLE. Therefore, cell therapy that can regenerate GABAergic neurons has been investigated as a potential treatment through transplantation of external stem cells or GABAergic progenitor cells⁴. In contrast to external cell therapy, using brain internal glial cells to regenerate functional GABAergic neurons may provide an alternative approach to treat TLE. One obvious advantage of in vivo cell conversion technology is to avoid the preparation of external cell bank that can be both costly and challenging in terms of maintaining cell homogeneity over many years. Another advantage is that local astrocytes tend to be converted into local neuronal identity to rescue local deficits.

Besides cell therapy and traditional anti-epileptic drug (AED) approach, gene therapy has also been investigated as an alternative approach for epilepsy in recent years⁴³. Transferring genes that can decrease neural network excitability such as channelrhodopsin-2 in GABAergic inhibitory neurons, modified muscarinic receptor hM4Di, engineered glutamate-gated Cl⁻ channel or K⁺ channel into excitatory neurons of epileptogenic foci have shown promise in suppressing seizure activity⁴⁴⁻⁴⁸. Compared to these classical gene therapies that often modify certain gene so that the expression level of a specific protein is increased or decreased, our NeuroD 1-based gene therapy is distinctly different. While said therapy does increase the expression level of NeuroD1, the NeuroD 1 protein expression *per se* is not an endpoint. The real endpoint of said NeuroD 1 gene therapy is to trigger astrocyte-to-neuron conversion, so that a new cell is generated. Therefore, said gene therapy is also a cell therapy, and the efficacy of said gene therapy is measured by how many functional new neurons can be generated, a very different concept from the other gene therapies. The advantage of this gene therapy-based cell therapy is the regeneration of functional GABAergic neurons and restoration of the excitatory-inhibitory circuits disrupted by seizure activities. The results demonstrate that introducing newly generated GABAergic neurons back into local hippocampal circuit can significantly inhibit the spontaneous recurrent seizures and rescue the deficit of cognitive and mood functions. Importantly, such local cell conversion technology can target a specific brain region without affecting the entire brain as experienced by systemic administration of AEDs. Moreover, the gene product NeuroD1 is an endogenous protein and its expression level will be gradually silenced due to the silencing of GFAP promoter in converted neurons, making it a relatively safe therapy.

It is worth noting that NeuroD1-mediated astrocyte-to-neuron conversion not only generates functional new neurons, but also protects the preexisting neurons, likely due to the effect of reducing SRS activity, thereby avoiding further neuronal death.

In one aspect, the present invention provides a method for treating temporal lobe epilepsy (TLE) in a subject, comprising administering a nucleic acid encoding a NeuroD 1 polypeptide to glial cells in hippocampus region of the subject, so that the glial cells express the NeuroD1 polypeptide and are converted into GABAergic neurons.

In one embodiment, the glial cells are located in CA1 region of the hippocampus region. In another embodiment, the glial cells are astrocytes, optionally, reactive astrocytes. In another embodiment, the glial cells are NG2 glial cells, oligodendrocytes, or microglial cells.

In one embodiment, the NeuroD1 polypeptide is a human NeuroD1 polypeptide. In another embodiment, the nucleic acid encoding the NeuroD 1 polypeptide is administered to the glial cells in the form of a viral vector, optionally, the viral vector is an adeno-associated viral vector. In another embodiment, the administration comprises direct injection into the hippocampus region.

In one embodiment, the nucleic acid encoding the NeuroD1 polypeptide is operably linked to a promoter sequence, preferably, the promoter is a tissue-specific promoter, more preferably, the promoter is a glial cell-specific promoter. In another embodiment, the promoter is an astrocyte-specific promoter. Preferably, the promoter is a glial fibrillary acidic protein (GFAP) promoter. More preferably, the promoter is a human glial fibrillary acidic protein (hGFAP) promoter. In another embodiment, the expression level of the nucleic acid encoding the NeuroD1 polypeptide gradually decreases in converted GABAergic neurons until it is not expressed in fully mature neurons.

In one embodiment, at least about 30%, 40%, 50%, 60%, 70%, 80%, 90%, 95% of the glial cells expressing the NeuroD1 polypeptide are converted into GABAergic neurons. In another embodiment, the GABAergic neurons are GAD67 positive. In another embodiment, the GABAergic neurons are positive for one or more of the following proteins: parvalbumin (PV), neuronal nitric oxide synthase (nNOS), somatostatin (SST), calretinin (CR), calbindin (CB), or cholecystokinin (CCK).

In another aspect, the present invention provides a method for forming GABAergic neurons in hippocampus region of a living mammalian brain, comprising administering a nucleic acid encoding a NeuroD1 polypeptide to glial cells located in the hippocampus region, so that the glial cells express the NeuroD 1 polypeptide and are converted into the GABAergic neurons.

In an embodiment, the mammal is a human. In another embodiment, the glial cells are located in CA1 region of the hippocampus region. In another embodiment, the glial cells are astrocytes, optionally, reactive astrocytes. In another embodiment, the glial cells are NG2 glial cells, oligodendrocytes, or microglial cells.

In one embodiment, the NeuroD1 polypeptide is a human NeuroD 1 polypeptide. In another embodiment, the nucleic acid encoding the NeuroD 1 polypeptide is administered to the glial cells in the form of a viral vector, optionally, the viral vector is an adeno-associated viral vector. In another embodiment, the administration comprises direct injection into the hippocampus region.

In one embodiment, the nucleic acid encoding the NeuroD1 polypeptide is operably linked to a promoter sequence. Preferably, the promoter is a tissue-specific promoter, and more preferably, the promoter is a glial cell-specific promoter. In another embodiment, the promoter is an astrocyte-specific promoter. Preferably, the promoter is a glial fibrillary acidic protein (GFAP) promoter. More preferably, the promoter is a human glial fibrillary acidic protein (hGFAP) promoter. In another embodiment, the expression level of the nucleic acid encoding the NeuroD1 polypeptide gradually decreases in converted GABAergic neurons until it is not expressed in fully mature neurons.

In one embodiment, at least about 30%, 40%, 50%, 60%, 70%, 80%, 90%, 95% of the glial cells expressing the NeuroD1 polypeptide are converted into GABAergic neurons. In another embodiment, the GABAergic neurons are GAD67 positive. In another embodiment, the GABAergic neurons are positive for one or more of the following proteins: parvalbumin (PV), neuronal nitric oxide synthase (nNOS), somatostatin (SST), calretinin (CR), calbindin (CB), or cholecystokinin (CCK).

In another aspect, the present invention provides use of a nucleic acid encoding a NeuroD 1 polypeptide in the manufacture of a medicament for treating temporal lobe epilepsy (TLE) in a subject, wherein the treatment comprises administering the nucleic acid encoding the NeuroD1 polypeptide to glial cells in hippocampus region of the subject, so that the glial cells express the NeuroD1 polypeptide and are converted into GABAergic neurons.

In one embodiment, the glial cells are located in CA1 region of the hippocampus region. In another embodiment, the glial cells are astrocytes, optionally, reactive astrocytes. In another embodiment, the glial cells are NG2 glial cells, oligodendrocytes, or microglial cells.

In one embodiment, the NeuroD1 polypeptide is a human NeuroD 1 polypeptide. In another embodiment, the nucleic acid encoding the NeuroD1 polypeptide is present in a viral vector, optionally, an adeno-associated viral vector.

In one embodiment, the nucleic acid encoding the NeuroD1 polypeptide is operably linked to a promoter sequence. Preferably, the promoter is a tissue-specific promoter, and more preferably, the promoter is a glial cell-specific promoter. In another embodiment, the promoter is an astrocyte-specific promoter. Preferably, the promoter is a glial fibrillary acidic protein (GFAP) promoter. More preferably, the promoter is a human glial fibrillary acidic protein (hGFAP) promoter. In another embodiment, the expression level of the nucleic acid encoding the NeuroD1 polypeptide gradually decreases in converted GABAergic neurons until it is not expressed in fully mature neurons.

In one embodiment, at least about 30%, 40%, 50%, 60%, 70%, 80%, 90%, 95% of the glial cells expressing the NeuroD1 polypeptide are converted into GABAergic neurons. In another embodiment, the GABAergic neurons are GAD67 positive. In another embodiment, the GABAergic neurons are positive for one or more of the following proteins: parvalbumin (PV), neuronal nitric oxide synthase (nNOS), somatostatin (SST), calretinin (CR), calbindin (CB), or cholecystokinin (CCK).

In another aspect, the present invention provides use of a nucleic acid encoding a NeuroD 1 polypeptide in the manufacture of a medicament for converting glial cells in hippocampus region of a living mammalian brain into GABAergic neurons, wherein the conversion comprises causing the glial cells to express the NeuroD1 polypeptide and to be converted into GABAergic neurons.

In one embodiment, the mammal is a human. In another embodiment, the glial cells are located in CA1 region of the hippocampus region. In another embodiment, the glial cells are astrocytes, optionally, reactive astrocytes. In another embodiment, the glial cells are NG2 glial cells, oligodendrocytes, or microglial cells.

In one embodiment, the NeuroD1 polypeptide is a human NeuroD 1 polypeptide. In another embodiment, the nucleic acid encoding the NeuroD1 polypeptide is present in a viral vector, optionally, an adeno-associated viral vector.

In one embodiment, the nucleic acid encoding the NeuroD1 polypeptide is operably linked to a promoter sequence. Preferably, the promoter is a tissue-specific promoter, and more preferably, the promoter is a glial cell-specific promoter. In another embodiment, the promoter is an astrocyte-specific promoter. Preferably, the promoter is a glial fibrillary acidic protein (GFAP) promoter. More preferably, the promoter is a human glial fibrillary acidic protein (hGFAP) promoter. In another embodiment, the expression level of the nucleic acid encoding the NeuroD1 polypeptide gradually decreases in converted GABAergic neurons until it is not expressed in fully mature neurons.

In one embodiment, at least about 30%, 40%, 50%, 60%, 70%, 80%, 90%, 95% of the glial cells expressing the NeuroD1 polypeptide are converted into GABAergic neurons. In another embodiment, the GABAergic neurons are GAD67 positive. In another embodiment, the GABAergic neurons are positive for one or more of the following proteins: parvalbumin (PV), neuronal nitric oxide synthase (nNOS), somatostatin (SST), calretinin (CR), calbindin (CB), or cholecystokinin (CCK).

In one aspect, the present invention provides a method for treating temporal lobe epilepsy (TLE) in a subject, comprising administering a NeuroD 1 polypeptide into the interior of glial cells in hippocampus region of the subject, so that the glial cells are converted into GABAergic neurons.

In another aspect, the present invention provides a method for forming GABAergic neurons in hippocampus region of a living mammalian brain, comprising administering a NeuroD1 polypeptide into the interior of glial cells located in the hippocampus region, so that the glial cells are converted into GABAergic neurons.

In one aspect, the present invention provides use of a NeuroD1 polypeptide in the manufacture of a medicament for treating temporal lobe epilepsy (TLE) in a subject, wherein the treatment comprises administering the NeuroD1 polypeptide into the interior of glial cells in hippocampus region of the subject, so that the glial cells are converted into GABAergic neurons.

In another aspect, the present invention provides use of a NeuroD1 polypeptide in the manufacture of a medicament for converting glial cells in hippocampus region of a living mammalian brain into GABAergic neurons, wherein the conversion comprises administering the NeuroD 1 polypeptide into the interior of the glial cells in hippocampus region of the subject, so that the glial cells are converted into GABAergic neurons.

### (I) Treatment Method

In a first aspect, the present invention provides a method for treating temporal lobe epilepsy (TLE) in a subject, comprising administering a nucleic acid encoding a NeuroD 1 polypeptide to glial cells in hippocampus region of the subject, so that the glial cells express the NeuroD1 polypeptide and are converted into GABAergic neurons.

GABA (γ-aminobutyric acid) mediates most of the fast inhibitory synaptic transmission in the brain. GABA is the main inhibitory neurotransmitter in the brain and is distributed in a variety of inhibitory interneurons and projection neurons. GABA content is higher in Purkinje cells in the superficial layers of the cerebral cortex, hippocampus and cerebellar cortex. GABAergic neuron degeneration is associated with epilepsy, Huntington's disease, tardive dyskinesia and sleep disorder and the like.

Deficits in GABAergic neurons are one of the main causes of TLE. In one embodiment, the subject is a human. In another embodiment, the mammal is a pet, such as a dog, a cat, etc.

Glial cells are another type of cells in the nervous system besides neurons, and together with neurons, form nervous tissue. In one embodiment, the glial cells are located in CA1 region of the hippocampus. In the other embodiment, the glial cells are located in CA3 region of the hippocampus. In another embodiment, the glial cells are located in DG region of the hippocampus.

The present invention also provides a method for treating temporal lobe epilepsy (TLE) in a subject, comprising administering a NeuroD1 polypeptide into the interior of glial cells in hippocampus region of the subject, so that the glial cells are converted into GABAergic neurons.

In an embodiment, the glial cells are astrocytes. In another embodiment, the glial cells are reactive astrocytes. Astrocyte activation is a specific manifestation of astrocyte plasticity, also known as reactive gliosis. Generation of reactive astrocytes is a common response of the central nervous system in many pathophysiological situations. Trauma, ischemia, hypoxia, infection, poisoning, radiation, immune diseases, etc. can all cause astrocyte activation. In an embodiment, the glial cells are non-reactive astrocytes. In an embodiment, no neuronal damage occurs around the astrocytes.

In an embodiment, the glial cells are NG2 glial cells, oligodendrocytes, or microglial cells. NG2 glial cells are also called oligodendrocyte progenitor cells (OPCs). In another embodiment, the glial cells are reactive NG2 glial cells, reactive oligodendrocytes, or reactive microglial cells. In another embodiment, the glial cells are non-reactive NG2 glial cells, non-reactive oligodendrocytes, or non-reactive microglial cells.

NeuroD1 is a bHLH preneuronal transcription factor involved in embryonic brain development and adult neurogenesis, which comprises a basic helix-loop-helix (bHLH) domain. The helix-loop-helix (HLH) domain is formed by two α-helices connected by a non-conservative loop region and is used for dimerization; and the basic domain mediates DNA binding. Binding of the basic domain to DNA requires HLH to first form dimers with other HLHs (for example, heterodimers with E47). The crystal structure of the E47-NeuroD1/Beta2 bHLH domain-DNA complex has been reported, which shows the amino acid positions in the bHLH domain that are critical for complex formation (*see* Antonella Longo et al., Biochemistry 2008, 47, 218 -229; Danile J. Dennis, Brain Research 1705 (2019) 48-65, the entire content of which is incorporated herein by reference).

In one embodiment, the NeuroD1 polypeptide is a human NeuroD1 polypeptide or a functional variant thereof. In another embodiment, the NeuroD1 polypeptide is a mouse NeuroD1 polypeptide or a functional variant thereof. In another embodiment, the NeuroD1 polypeptide is a NeuroD1 polypeptide derived from another species (e.g., rat) or a functional variant thereof.

In one embodiment, the nucleic acid encoding the NeuroD1 polypeptide is administered to the glial cells in the form of a viral vector. In an embodiment, the viral vector is an adeno-associated virus (AAV), for example, an adeno-associated virus serotype 2 viral vector, an adeno-associated virus serotype 5 viral vector, or an adeno-associated virus serotype 9 viral vector. In another embodiment, the viral vector is an adenovirus. In another embodiment, the viral vector is a retrovirus. In another embodiment, the viral vector is a lentivirus.

In another embodiment, the nucleic acid encoding the NeuroD1 polypeptide (e.g., a plasmid) is administered to the glial cells in the form of a non-viral vector, such as an exosome, a liposome, or a nanoparticle. In another embodiment, gene editing technologies such as CRISPR-mediated gene editing can be used to induce controlled expression of endogenous NeuroD 1 factors.

In another embodiment, the NeuroD1 polypeptide is administered directly to the glial cells located in the hippocampus region. For example, through exosomes, liposomes or nanoparticles, or by the fusing NeuroD1 polypeptide with a membrane-penetrating peptide, the NeuroD 1 polypeptide can directly penetrate the membrane and enter cells.

The dosages administered above are therapeutically effective dosages. Those skilled in the art understand that the dosages administered herein can be adjusted according to actual conditions based on various factors, such as vector selection, cells, general condition of the subject to be treated, degree of treatment/improvement sought, route of administration, mode of administration, etc.

The dosage may further include a carrier (water, saline, ethanol, glycerol, lactose, sucrose, calcium phosphate, gelatin, dextran, agar, pectin, peanut oil, sesame oil, etc.), diluent, pharmaceutically acceptable carrier (e.g. phosphate buffered saline), pharmaceutically acceptable excipients and/or other compounds known in the art. The dosage may further comprise one or more pharmaceutically acceptable salts, such as inorganic acid salts, such as hydrochlorides, hydrobromides, phosphates, sulfates, etc.; and salts of organic acids, such as acetates, propionate, malonate, benzoate, etc. In addition, auxiliary substances such as wetting or emulsifying agents, pH buffer substances, gels or gelling materials, etc. may also be present. In addition, one or more other conventional pharmaceutical ingredients, such as preservatives, wetting agents, suspending agents, surfactants, antioxidants, anti-caking agents, fillers, chelating agents, coating agents, chemical stabilizers, etc. can also be present. Pharmaceutically acceptable excipients are discussed in detail in REMINGTON'S PHARMACEUTICAL SCIENCES (Mack Pub. Co., N.J. 1991), which is incorporated herein by reference.

In one embodiment, the administration comprises injection directly into the hippocampus region, such as by intracranial injection. In another embodiment, the administration comprises intraperitoneal, intravenous, intranasal, or oral administration, so as to target a region of interest, such as the hippocampus. Alternatively, other means such as liposomes may be used to target the substance to be administered across the blood-brain barrier to the region of interest, such as the hippocampus region.

In one embodiment, the nucleic acid encoding the NeuroD1 polypeptide is operably linked to a promoter sequence. In an embodiment, the promoter is a constitutive promoter, including but not limited to a CMV promoter, a CAG promoter, a CBG promoter, an EF1a promoter, an UbC promoter, a PGK promoter, etc. In another embodiment, the promoter is an inducible promoter, including but not limited to a TRE promoter, a c-fos promoter. In another embodiment, the promoter is a tissue-specific promoter.

In an embodiment, the promoter is a glial cell-specific promoter. In an embodiment, the promoter is an astrocyte promoter, including but not limited to a GFAP promoter, a Cst3 promoter, a Cx30 promoter. In another embodiment, the promoter is an oligodendrocyte promoter, including but not limited to a PDGFRA promoter, an olig2 promoter, a NG2 promoter. In another embodiment, the promoter is a microglial cell promoter, including but not limited to CX3CR1 and IBa1 promoters. In another embodiment, the promoter is a high-abundance promoter, a medium-abundance promoter, or a low-abundance promoter. Types of high abundance promoters, medium abundance promoters or low abundance promoters are well known in the art. When glial cells are converted into neurons, the glial cell-specific promoter is silenced, and the NeuroD1 polypeptide is no longer expressed in the converted neurons.

In an embodiment, the promoter is an astrocyte-specific promoter. Preferably, the promoter is a glial fibrillary acidic protein (GFAP) promoter. More preferably, the promoter is a human glial fibrillary acidic protein (hGFAP) promoter. When astrocytes are converted into neurons, the astrocyte-specific promoter is silenced, and the NeuroD1 polypeptide is no longer expressed in the converted neurons.

In addition to the nucleic acid encoding the NeuroD 1 polypeptide and the promoter, the vector may also comprise regulatory elements operably linked to the nucleic acid encoding the NeuroD1 polypeptide, including an enhancer sequence, a response element, a signal peptide, an internal ribosome entry sequence, a polyadenylation signal, a terminator or an inducible element, etc.

In one embodiment, the expression level of the nucleic acid encoding the NeuroD1 polypeptide gradually decreases in converted GABAergic neurons until it is not expressed in fully mature neurons.

In an embodiment, at least about 10%, 20%, 30%, 35%, 40%, 45%, 50%, 55%, 60%, 65%, 70%, 75%, 80%, 85%, 90 %, 95% of the glial cells expressing the NeuroD1 polypeptide are converted into GABAergic neurons. In one embodiment, at least about 21%, 22%, 23%, 24%, 25%, 26%, 27%, 28%, 29%, 30% of the glial cells expressing the NeuroD1 polypeptide are converted into GABAergic neurons. In one embodiment, at least about 31%, 32%, 33%, 34%, 35%, 36%, 37%, 38%, 39%, 40% of the glial cells expressing the NeuroD1 polypeptide are converted into GABAergic neurons. In one embodiment, at least about 41%, 42%, 43%, 44%, 45%, 46%, 47%, 48%, 49%, 50% of the glial cells expressing the NeuroD1 polypeptide are converted into GABAergic neurons. In one embodiment, at least about 51%, 52%, 53%, 54%, 55%, 56%, 57%, 58%, 59%, 60% of the glial cells expressing the NeuroD1 polypeptide are converted into GABAergic neurons. In another embodiment, at least about 61%, 62%, 63%, 64%, 65%, 66%, 67%, 68%, 69%, 70% of the glial cells expressing the NeuroD1 polypeptide are converted into GABAergic neurons. In another embodiment, at least about 71%, 72%, 73%, 74%, 75%, 76%, 77%, 78%, 79%, 80% of the glial cells expressing the NeuroD1 polypeptide are converted into GABAergic neurons. In another embodiment, at least about 81%, 82%, 83%, 84%, 85%, 86%, 87%, 88%, 89%, 90% of the glial cells expressing the NeuroD1 polypeptide are converted into GABAergic neurons. In another embodiment, at least about 91%, 92%, 93%, 94%, 95%, 96%, 97%, 98%, 99%, 100% of the glial cells expressing the NeuroD1 polypeptide are converted into GABAergic neurons.

In one embodiment, the GABAergic neurons are GAD67 positive. The GAD67 is a marker of the GABAergic neurons.

In another embodiment, the GABAergic neurons present (one or more) GABAergic neurons subtype markers, such as parvalbumin (PV) positive, neuronal nitric oxide synthase (nNOS) positive, somatostatin (SST) positive, calretinin (CR) positive, calbindin (CB) positive, or cholecystokinin (CCK) positive.

The present invention also encompasses a method for treating temporal lobe epilepsy (TLE) in a subject, comprising administering a pharmaceutical composition (comprising a nucleic acid encoding a NeuroD1 polypeptide or a NeuroD1 polypeptide) to glial cells in hippocampus region of the subject, so that the glial cells are converted into GABAergic neurons. The pharmaceutical composition may further comprise an additional cytokine (e.g., a transcription factor), a small molecule compound, or a combination thereof.

Other relevant embodiments can be found in CN104870634A, which is incorporated herein by reference.

In a second aspect, the present invention provides a method for forming GABAergic neurons in hippocampus region of a living mammalian brain, comprising administering a nucleic acid encoding a NeuroD1 polypeptide to glial cells located in the hippocampus region, so that the glial cells express the NeuroD 1 polypeptide and are converted into GABAergic neurons.

The present invention also provides a method for forming GABAergic neurons in hippocampus region of a living mammalian brain, comprising administering a NeuroD 1 polypeptide into the interior of glial cells located in the hippocampus region, so that the glial cells are converted into the GABAergic neurons.

In one embodiment, the mammal is a human. In another embodiment, the mammal is a pet, such as a dog, a cat, etc.

The present invention also encompasses a method for forming GABAergic neurons in hippocampus region of a living mammalian brain, comprising administering a pharmaceutical composition (comprising a nucleic acid encoding a NeuroD1 polypeptide or a NeuroD1 polypeptide) to glial cells in the hippocampus region of the subject, so that the glial cells are converted into the GABAergic neurons. The pharmaceutical composition may further comprise an additional cytokine (e.g., a transcription factor), a small molecule compound, or a combination thereof.

For other specific embodiments of the second aspect, please refer to the description of the first aspect.

### (II) Pharmaceutical Use

In one aspect, the present invention provides use of a nucleic acid encoding a NeuroD 1 polypeptide in the manufacture of a medicament for treating temporal lobe epilepsy (TLE) in a subject, wherein the treatment comprises administering the nucleic acid encoding the NeuroD1 polypeptide to glial cells in hippocampus region of the subject, so that the glial cells express the NeuroD1 polypeptide and are converted into GABAergic neurons.

The present invention also provides use of a NeuroD 1 polypeptide in the manufacture of a medicament for treating temporal lobe epilepsy (TLE) in a subject, wherein the treatment comprises administering the NeuroD1 polypeptide into the interior of glial cells in hippocampus region of the subject, so that the glial cells are converted into GABAergic neurons.

The present invention also encompasses use of a pharmaceutical composition (comprising a nucleic acid encoding a NeuroD 1 polypeptide or a NeuroD1 polypeptide) in the manufacture of a medicament for the treatment of temporal lobe epilepsy (TLE) in a subject, wherein the treatment comprises administering the pharmaceutical composition to glial cells in hippocampus region of the subject, so that the glial cells are converted into GABAergic neurons. The pharmaceutical composition may further comprise an additional cytokine (e.g., a transcription factor), a small molecule compound, or a combination thereof.

In another aspect, the present invention provides use of a nucleic acid encoding a NeuroD 1 polypeptide in the manufacture of a medicament for converting glial cells in hippocampus region of a living mammalian brain into GABAergic neurons, wherein the conversion comprises causing the glial cells to express the NeuroD 1 polypeptide and to be converted into the GABAergic neurons.

The present invention also provides use of a NeuroD 1 polypeptide in the manufacture of a medicament for converting glial cells in hippocampus region of a living mammalian brain into GABAergic neurons, wherein the conversion comprises administering the NeuroD1 polypeptide into the interior of glial cells in the hippocampus region of the subject, so that the glial cells are converted into the GABAergic neurons.

The present invention also encompasses use of a pharmaceutical composition (comprising a nucleic acid encoding a NeuroD 1 polypeptide or a NeuroD1 polypeptide) in the manufacture of a medicament for converting glial cells in hippocampus region of a living mammalian brain into GABAergic neurons, wherein the conversion comprises administering the pharmaceutical composition to glial cells in hippocampus region of the subject, so that the glial cells are converted into the GABAergic neurons. The pharmaceutical composition may further comprise an additional cytokine (e.g., a transcription factor), a small molecule compound, or a combination thereof.

Specific embodiments in the (II) Pharmaceutical Use section may refer to the relevant specific embodiments described in (I) Treatment Method.

The present invention also provides other technical solutions equivalent to the treatment method or the pharmaceutical use, and these solutions are also within the scope of the present invention.

### Definition

Unless defined otherwise, all technical and scientific terms used herein have the same meaning as commonly understood by those of ordinary skill in the art to which the present invention belongs. For the purposes of the present invention, the following terms are defined to be consistent with their commonly understood meanings in the art.

As used herein and in the appended claims, the singular forms "a", "an", "another", and "the" include plural referents unless the context clearly indicates otherwise.The terms "comprise", "comprising", "having", "e.g.", etc. are intended to convey inclusion rather than limitation unless the context clearly indicates otherwise.

The term "GABAergic neurons" also includes cells with the phenotype of GABAergic neurons that are converted from other cells, including neurons in the process of conversion and fully mature neurons. The phenotype of GABAergic neurons includes one or more of the following: GABAergic neuronal morphology, expression of one or more GABAergic neuronal markers, electrophysiological features of the GABAergic neurons, synaptic formation, and realse of neurotransmitters.

NeuroD1 is a bHLH preneuronal transcription factor involved in embryonic brain development and adult neurogenesis, *see* Cho, J. H. et al., Mol. Neurobiol., 30:35-47, 2004; Kuwabara, T. et al., Nature Neurosci., 12:1097-1105, 2009; and Gao, Z. et al., Nature Neurosci., 12:1090-1092, 2009. NeuroDl is expressed late in development-primarily in the nervous system-and is involved in neuronal differentiation, maturation and survival.

The term "NeuroD1 polypeptide" includes wild-type NeuroD1 polypeptides from different species, functional fragments thereof, or functional variants thereof. The functional variant still has all or part of the activity of the wild-type NeuroD1 polypeptide. The functional variant may be formed by insertion, deletion, substitution of one or more amino acids, or a combination thereof, based on a wild-type polypeptide or a functional fragment thereof, including naturally occurring genetic variations (e.g., single nucleotide polymorphism) and variations resulting from the recombination methods. The NeuroD1 polypeptide may be a human NeuroD1 polypeptide, for example, having the amino acid sequence set forth in SEQ ID NO: 1. The NeuroD1 polypeptide may be a mouse NeuroD 1 polypeptide, for example, having the amino acid sequence set forth in SEQ ID NO: 3. The functional variant may have at least 60%, 65%, 70%, 75%, 80%, 85%, 90%, 91%, 92%, 93%, 94%, 95%, 96%, 97%, 98%, 99% identity to the amino acid sequence set forth in SEQ ID NO: 1 or SEQ ID NO: 3, and have activity (fully or partially). Relevant sequence information can be found in CN104870634A, which is incorporated herein by reference. The functional variant may be obtained by making one or more of the following mutations based on the wild-type sequence (e.g., SEQ ID NO: 1 or SEQ ID NO: 3): insertion (extension) at either or both ends of the sequence, deletions (truncation) at either or both ends of the sequence, substitution of one or more amino acids, insertion within the sequence, deletion within the sequence. The substitution of amino acids is a conservative substitution of non-critical amino acids without affecting the activity or still retaining part of the activity. The non-critical amino acids may be contemplated being located, for example, outside the bHLH domain, for example, at non-conservative amino acid positions within the bHLH domain, for example, in non-conservative loops within the bHLH domain. The number of non-critical amino acid substitutions may be, for example, less than 20, such as 19, 18, 17, 16, 15, 14, 13, 12, 11, 10, 9, 8, 7, 6, 5, 4, 3, 2, 1. An additional sequence may be inserted or a portion of the sequence may be deleted at either or both ends of the sequence without affecting the activity. The length of the inserted or deleted sequence may be, for example, less than 20, such as 19, 18, 17, 16, 15, 14, 13, 12, 11, 10, 9, 8, 7, 6, 5, 4, 3, 2, or 1. One or more amino acids, for example 1, 2, 3, 4 or 5, can be inserted or deleted in the loop region.

"Sequence identity" between two polypeptide or nucleic acid sequences represents the percentage of the number of residues that are identical between the sequences in the total number of the residues, and the calculation of the total number of the residues is determined based on the mutation type. The mutation type includes insertion (extension) at either or both ends of A sequence, deletion (truncation) at either or both ends of a sequence, substitution/replacement of one or more amino acids/nucleotides, insertion within a sequence, deletion within a sequence. Taking polypeptides as an example (the same goes for nucleotides), if the mutation type is one or more of the following: substitution/replacement of one or more amino acids/nucleotides, insertion within the sequence, and deletion within the sequence, then the total number of residues is calculated with the larger one of the molecules compared. If the mutation type further includes an insertion (extension) at either or both ends of a sequence or a deletion (truncation) at either or both ends of a sequence, then the number of amino acids inserted or deleted at either or both ends is not included in the total number of residues (in the case of NeuroD1 polypeptide, the number of insertions or deletions at both ends is less than 20). In calculating percentage identity, the sequences being compared are aligned in a manner that produces the greatest match between the sequences, with gaps in the alignment (if any) being resolved by a specific algorithm.

Non-critical amino acids can be conservatively substituted without affecting the normal function of the protein, such as NeuroD1 polypeptide. Conservative substitution means the substitution of an amino acid with a chemically or functionally similar amino acid. It is well known in the art to provide conservative substitution tables for similar amino acids. For example, in some embodiments, the amino acid groups provided in Tables 1-3 are regarded as conservative substitutions of each other.

**Table 1 Selected groups of amino acids that are regarded as conservative substitutions for each other in certain embodiments**

| | |
|---|---|
| Acidic residues | D and E |
| Basic residues | K, R and H |
| Hydrophilic uncharged residues | S, T, N and Q |
| Aliphatic uncharged residues | G, A, V, L and I |
| Non-polar uncharged residues | C, M and P |
| Aromatic residues | F, Y and W |

**Table 2 Aother selected groups of amino acids that are regarded as conservative substitutions for each other in certain embodiments**

| | |
|---|---|
| Group 1 | A, S and T |
| Group 2 | D and E |
| Group 3 | N and Q |
| Group 4 | R and K |
| Group 5 | I, L and M |
| Group 6 | F, Y and W |

**Table 3 Aother selected groups of amino acids that are considered conservative substitutions for each other in certain embodiments**

| | |
|---|---|
| Group A | A and G |
| Group B | D and E |
| Group C | N and Q |
| Group D | R, K and H |
| Group E | I, L, M, V |
| Group F | F, Y and W |
| Group G | S and T |
| Group H | C and M |

The term "amino acid" means twenty common naturally occurring amino acids. Naturally occurring amino acids include alanine (Ala; A), arginine (Arg; R), asparagine (Asn; N), aspartic acid (Asp; D), cysteine (Cys; C); glutamic acid (Glu; E), glutamine (Gln; Q), glycine (Gly; G), histidine (His; H), isoleucine (Ile; I), leucine ( Leu; L), lysine (Lys; K), methionine (Met; M), phenylalanine (Phe; F), proline (Pro; P), serine (Ser; S), threonine (Thr; T), tryptophan (Trp; W), tyrosine (Tyr; Y), and valine (Val; V).

The term "operably linked" refers to the position of the regulatory element relative to the nucleic acid in the vector that allows or promotes expression of the encoded polypeptide. For example, the GFAP promoter is operably linked to a nucleic acid encoding a NeuroD1 polypeptide to drive its transcription in astrocytes.

As used herein, the term "conversion/converted" is used to describe the effect of expressing a NeuroD1 polypeptide, which causes glial cells to become GABAergic neurons or cells with a GABAergic neuronal phenotype.

As used herein, the term "treating/treatment" means alleviating, inhibiting or ameliorating a neurological condition or a symptom thereof, as well as preventing a neurological condition or a symptom thereof, including but not limited to therapeutic and/or preventive treatment.

It should be understood that one, some, or all of the features of each embodiment described herein may be combined to form other embodiments of the present invention. These and other aspects of the present invention would become obvious to those skilled in the art.

Although the present invention has been described with reference to its specific embodiments, those skilled in the art will understand that various changes may be made and equivalent solutions may be replaced without departing from the true spirit and scope of the present invention. It is clear to those skilled in the art that other appropriate modifications and transformations can be made to the methods described herein using appropriate equivalent solutions without departing from the scope of the embodiments disclosed herein. Certain embodiments having been described in detail, and they will be understood more clearly by reference to the following examples, which are included for illustrative purposes only and are not intended for limiting.

### EXAMPLES

### 1. Result

### 1.1 NeuroD1 converts hippocampal astrocytes into GABAergic neurons in rat TLE model

GABAergic neuronal loss is one of the major hallmarks of TLE. First, a TLE model was established in rats by injecting pilocarpine (350 mg/kg, i.p.) into adult Sprague Dawley rats as reported before²⁹. It was found that 94.3% (99 in 105 rats) of the rats experienced acute seizures (≥3, Racine scale), and 86.9% (86 in 99 rats) of the rats survived after seizure. At 7 days after status epilepticus (SE), one group of rats (36 in total without viral injection) were video-EEG monitored for ~27 days, and 77.8% of them (28 in 36 rats) developed SRS, similar to previous studies³⁰. Timm staining revealed Mossy fiber sprouting in SRS rats, but not in rats without SRS (Fig. 1). GAD67 and PV staining found a significant reduction of GABAergic neurons at 5 weeks after induction of TLE (Fig. 2a-d). These results demonstrate successful establishment of a rat TLE model with typical neuropathological features as reported before.

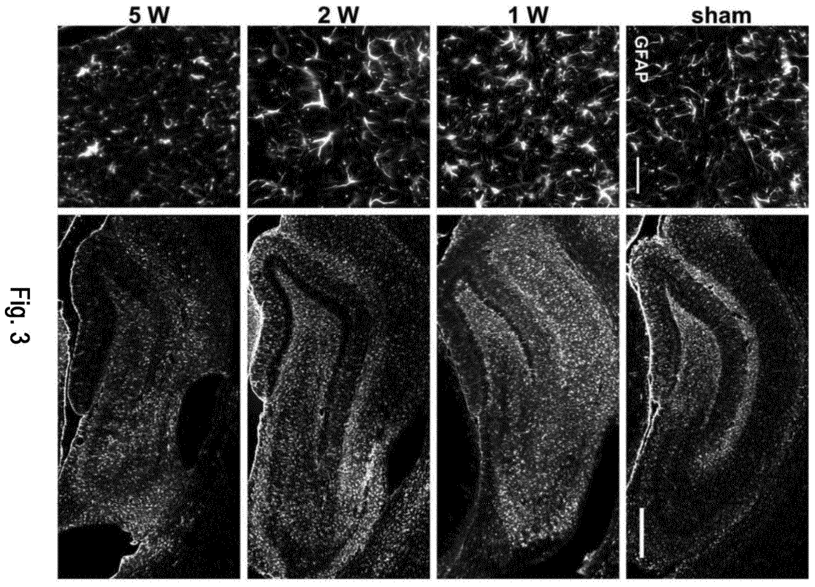

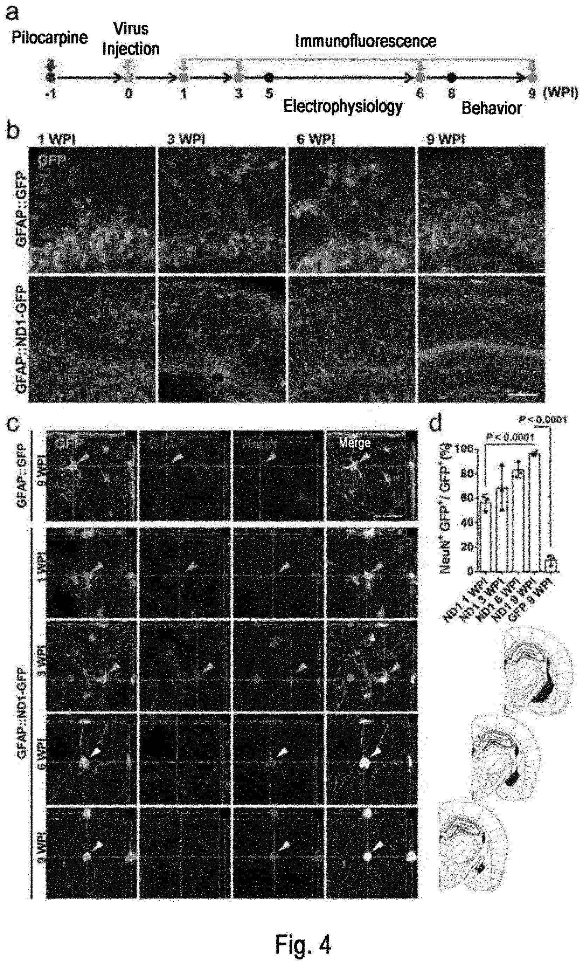

After successful establishment of the rat TLE model, adeno-associated virus 9 (AAV9) was employed to express NeuroD1 in hippocampal astrocytes under the control of an astrocyte-specific promoter (human glial fibrillary acidic protein (hGFAP)) (hGFAP::NeuroD1-P2A-GFP). Astrocytes became reactive (entered a stress state) after induction of TLE, as confirmed by GFAP immunostaining (Fig. 3). AAV9 NeuroD1 was injected bilaterally into the hippocampus (3 µl each side) at 1 week after SE to target the reactive astrocytes for conversion (see Fig. 4a for experimental design). As side-by-side control, expressing green fluorescent protein (GFP) alone without NeuroD1 (hGFAP::GFP) was employed to assess the difference between NeuroD1-GFP and GFP control. Fig. 4b illustrates that the AAV injection resulted in robust viral infection in the hippocampus of TLE rats when assessed at 1, 3, 6 and 9 weeks post viral injection (WPI). Fig. 5 illustrates that the viral infected cells were evenly distributed throughout the adult hippocampus. However, there is an obvious difference in the morphology of GFP-infected cells versus the NeuroD 1-GFP-infected cells throughout 1-9 weeks after viral infection: the GFP-infected cells in the control group showed consistent astrocytic morphology without much change within 9 weeks (Fig. 4b, top row), whereas NeuroD 1-GFP-infected cells showed clear morphological changes starting from 1 WPI and displaying neuronal morphology by 9 WPI (Fig. 4b, bottom row). High power orthogonal images in Fig. 4c provide better illustration that in GFP control group (9 WPI, top row), most of the GFP-infected cells were typical astrocytes, colocalizing with the astrocytic marker GFAP but not with neuronal marker NeuN. In contrast, in NeuroD 1-GFP group, while the NeuroD 1-infected cells did show GFAP immunostaining at 1 WPI and 3 WPI, suggesting that they were originated from GFAP+ astrocytes, the majority of NeuroD1-infected cells gradually transformed into neuron-like cells by 6 WPI and 9 WPI (Fig. 4c). Quantitatively, at 1 WPI, about half of the NeuroD1-GFP-labeled cells expressed both GFAP and NeuN (Fig. 4c, 56.5 ± 6.8%, 3 rats), indicating a transitional stage between astrocytes and neurons during early conversion process¹². At 3 WPI, many NeuroD1-GFP-infected cells started to lose GFAP signal and began to express NeuroD1 (68.1 ± 18.3%, 3 rats), with only a small portion coexpressing GFAP and NeuN (Fig. 4c, 3 WPI). At 6 WPI, most of the NeuroD1-GFP-infected cells showed neuronal morphology and only expressed NeuN (83.3 ± 6.4%, 3 rats) without GFAP (Fig. 4c, 6 WPI). By 9 WPI, almost all of the NeuroD1-GFP-infected cells showed mature neuronal morphology with long extensive neuronal protrusion, and expressed NeuN (Fig. 4c, 9 WPI). Quantitative analysis showed a steady increase of NeuN+ cells among the NeuroD1-GFP-infected cells from 1 to 9 WPI, with neuronal conversion efficiency reaching 96.0 ± 1.7% at 9 WPI (Fig. 4d, quantified region shown in red below the bar graph, n = 4 rats). We also examined whether NeuroD1 protein could be detected in the NeuroD1-GFP-infected cells, and found that most of the NeuroD 1-GFP-infected cells were labeled with NeuroD 1 at 1 WPI (Fig. 6a, b, 82.3 ± 5.9%, 4 rats), but NeuroD1 signal was rarely detectable by 9 WPI when astrocyte-converted neurons have matured and therefore the GFAP promoter has been silenced in mature neurons. Moreover, to further investigate whether NeuroD1-converted neurons are genuinely coming from astrocytes, we performed triple immunostaining (Fig. 6c) of GFP, NeuN, and Sox9 (another glial marker that would not be detected in neurons of the adult mammalian brains). Interestingly, among NeuroD1-GFP-infected cells (green) in the rat hippocampus, we detected clear co-localization of NeuN (red) with SOX9 (magenta, Fig. 6c), confirming that the astrocyte-converted neurons kept certain astrocytic properties during the early phase of conversion. The above results indicate that transient expression of NeuroD1 in hippocampal reactive astrocytes of TLE rats can gradually convert astrocytes into neurons.

Immunostaining for GAD67, a GABAergic neuronal marker, was performed in converted neurons from TLE rat hippocampus (Fig. 7). Surprisingly, unlike previous findings in the cortex¹², the majority of NeuroD 1-GFP-converted neurons in the TLE rat hippocampus were immunopositive for GAD67 (Fig. 7a, top row, quantified in Fig. 7b, 81.7 ± 5.8%, 4 rats). Additional immunostaining with interneuron subtype markers further revealed that subsets of NeuroD 1-GFP-converted neurons were immunopositive for parvalbumin (PV, 33.2 ± 4.1%, 4 rats), neuronal nitric oxide synthase (nNOS, 25.6 ± 7.7%, 5 rats), somatostatin (SST, 24.7 ± 3.7%, 5 rats), as well as calretinin (CR, 42.6 ± 17.1%, 3 rats), calbindin (CB, 26.4 ± 4.6%, 3 rats), or cholecystokinin (CCK, 21.3 ± 10.1%, 3 rats) (Fig. 7a, quantified in Fig. 7b). Together, these experiments demonstrate that in TLE rat model, hippocampal astrocytes can be directly converted into GABAergic neurons.

The high percentage of GABAergic neurons converted from hippocampal astrocytes in TLE rats was an unexpected finding, because earlier work in the mouse cortex found that the majority of cortical astrocyte-converted neurons in an ischemic stroke model was glutamatergic neurons¹². Side-by-side comparison experiments were performed to investigate the subtypes of converted neurons induced by the same AAV9 hGFAP::NeuroD1 vector in the cortex versus hippocampus (CA1 and DG), and in rats with or without TLE. As shown in Fig. 8a, while GAD67+ neurons were detectable in both hippocampus and cortex, quantified data showing higher percentage of GABAergic neurons in the CA1 region and lower percentage in the cortical region (Fig. 8b). Interestingly, TLE model itself also showed a significant impact on cell fate after conversion: the percentage of GABAergic neurons in CA1 region was higher in rats with TLE (72.5 ± 8.2%, 3 rats) than those without TLE (48.2 ± 9.0%, 4 rats), suggesting that the status of astrocytes with or without injury may affect its cell fate after conversion. The percentage of GABAergic neurons in the cortex and dentate gyrus (DG) was much lower than that in the CA1 region with or without TLE (Fig. 8b; without TLE: DG, 27.4 ± 3.7%, n = 4 rats; Cortex, 23.9 ± 4.5%, n = 3 rats; with TLE: DG, 33.6 ± 3.2%, n = 4 rats, Cortex, 18.9 ± 4.6%, n = 3 rats).

To exluce the possibility that NeuroD 1-converted neurons come from the adult neural stem cells in the DG, immunostaining of Prox1, a protein specifically expressed in DG granule cells but not in other hippocampal regions, was performed. As expected, it was found that Prox1 staining was indeed limited in the DG region (Fig. 9a). Among the NeuroD1-converted neurons, there were only a few Prox1-positive cells in the DG region and rarely in the CA1 region (Fig. 9b). Quantitative analysis showed that the number of Prox1-positive cells was very high in the DG (-800 cells/mm²) but very low in the CA1 region (Fig. 9c, first two bars). Among NeuroD 1-converted neurons, a few Prox1⁺ cells were found in the DG area but rarely in the CA1 region (Fig. 9c, GFP⁺&Prox1⁺ cells in DG: 11.6 ± 4.6/mm²; GFP⁺&Prox1⁺ cells in CA1: 2.4 ± 0.8/mm², P < 0.03, n = 3 rats). These results suggest that the newly generated neurons in the CA1 region are converted from astrocytes by NeuroD1, not from the adult neural stem cells in the DG.

### 1.2 Rescue of neuronal loss after AAV NeuroD1-treatment

We next investigated whether NeuroD1 gene therapy could rescue neuronal loss in the TLE rat hippocampus (Fig. 10). Fig. 10a illustrates typical immunostaining of GABAergic markers, including GAD67, PV, nNOS, SST, and NeuN, in non-TLE rats (sham), TLE rats, and TLE rats treated with NeuroD1 gene therapy (Fig. 10a). Quantitative analysis (Fig. 10b) showed that, the density of GAD67+ neurons in the CA1 region of TLE rats (36.7 ± 13.3 cells/mm², 5 rats) was significantly lower than that of sham rats (55.0 ± 3.4 cells /mm², 5 rats; P < 0.02). However, at 9 weeks after injection of NeuroD 1 AAV9, the NeuroD1-treated TLE rats showed a significant increase of GAD67+ neurons in the CA1 region (59.2 ± 7.4 cells/mm² in group, 5 rats; P < 0.006 compared to the TLE rats, one-way ANOVA followed with Tukey post hoc test). Previous studies have shown that PV⁺ neuronal density was impaired by SRS³³. Consistently, it was found that the PV⁺ neuronal density in the CA1 region of TLE rats (17.1 ± 5.5 cells/mm², 5 rats) was about half of that observed in sham rats (30.5 ± 3.6 cells/mm², 3 rats, P < 0.002, one-way ANOVA followed with Tukey post hoc test) (Fig. 10c). However, at 9 weeks after NeuroD1-treatment, the PV⁺ neuron density in the CA1 region of TLE rats was rescued to a level comparable to that of sham rats (Fig. 10c, NeuroD1 30.4 ± 4.3 cells/mm², 5 rats, P < 0.002). Similarly, TLE rats showed a decrease in nNOS⁺ (nNOS⁺, 20.2 ± 7.4 cell/mm², 5 rats) compared to sham rats (nNOS⁺, 32.9 ± 4.6 cell/mm², 5 rats, P < 0.03), but rescued after NeuroD1-treatment (Fig. 10d, nNOS⁺, 31.0 ± 6.7 cell/mm², 5 rats, P < 0.05 compared to TLE, one-way ANOVA followed with Tukey post hoc test). The SST⁺ neuron density also showed similar trend but did not reach statistical significance (Fig. 10e, Sham, 24.0 ± 7.9 cell/mm², TLE, 13.0 ± 8.4 cell/mm², NeuroD1, 20.8 ± 5.5 cell/mm², 5 animals in each group, P = 0.09, one-way ANOVA). Furthermore, the total neuronal density (NeuN+ cells) in the CA1 region was also significantly decreased in the TLE rats at 10 weeks after SE (Fig. 10f, sham: 561 ± 66.3 cells/mm², 5 rats; TLE: 390 ± 42.1 cells/mm², 5 rats, P < 0.002, one-way ANOVA followed with Tukey post hoc test). However, following NeuroD1-treatment, the total neuronal density showed a significant increase (Fig 10f, 534 ± 43.3 cells/mm², 5 rats, P < 0.001 compared to TLE, one-way ANOVA followed with Tukey post hoc test). In addition to neuronal rescue, the impact of NeuroD1 conversion on the number of astrocytes was also examined since astrocytes play important roles in brain functions³⁴. Specifically, we tested whether converting astrocytes into neurons might deplete astrocytes in the NeuroD 1-infected areas. The results showed that the intensity of astrocytic protein GFAP in the CA1 region was not different between the sham rats and TLE rats at 9 weeks after viral injection with or without NeuroD1 treatment (Fig. 11a, b, one-way ANOVA, P = 0.95). Similarly, quantification of the density of SOX9 also revealed no significant changes in astrocyte number among different groups with or without NeuroD1 treatment (Fig. 11a, c, one-way ANOVA, P = 0.51, 9 weeks after viral injection), consistent with the fact that astrocytes can proliferate for self-renewal³⁵. Together, the results suggest that NeuroD1-mediated astrocyte-to-neuron conversion can significantly rescue neuronal loss induced by TLE in the rat hippocampus region.

### 1.3 Functional characterization of NeuroD1-converted neurons in the TLE hippocampus

To test whether the NeuroD1-converted neurons in TLE rats were functional, whole-cell recordings were performed in acute hippocampal slices at 5-6 WPI. In GFP control group, none of the GFP-infected cells showed any action potential firing nor any postsynaptic events from 1 to 6 WPI (Fig. 12a). In contrast, NeuroD1-infected cells started to show single action potential as well as some spontaneous synaptic events at 3 WPI (Fig. 12b). By 6 WPI, essentially all the NeuroD1-GFP-converted neurons (27/27) were able to generate action potentials (AP) and many more synaptic events were recorded (Fig. 12b). Quantitative data showed that the AP frequency recorded from NeuroD 1-converted neurons at 6 WPI was similar to that recorded from sham rats (Fig. 12c, d). Similarly, the spontaneous postsynaptic currents (sPSCs) showed gradual increase from 1 to 6 WPI in NeuroD1-GFP-infected cells (Fig. 12e). During whole-cell recordings, recorded neurons were marked with biocytin (0.2%) *for post hoc* immunostaining (Fig. 12f-i, bottom panel; 6 WPI). Triple labeling (GFP, biocytin and GAD67) confirmed that 88.5% (Fig. 12k, 23 in 26 neurons) of recorded NeuroD 1-GFP-converted neurons expressed GAD67. In addition, the AP firing patterns from all recorded neurons (57 cells) were categorized into four subtypes: regular spiking (Fig. 12f, firing with adaptation, n = 16 cells, 28.1%), irregular spiking (Fig. 12g, n = 14 cells, 24.6%), single spike (Fig. 5h, less than 5 APs in 1 second, n = 12 cells, 21%), and fast spiking (Fig. 12i, firing without adaptation, n = 15 cells, 26.3%). The pie chart in Fig. 12j illustrates the proportion of each firing pattern. For the fast spiking neurons, we performed parvalbumin (PV) immunostaining and confirmed that the converted fast spiking neurons were PV⁺ interneurons (Fig. 12i). Taken together, these findings suggest that in the TLE rat hippocampus, NeuroD1-converted neurons show typical features of interneurons and can integrate into functional neural circuits by establishing synaptic connections with other neurons.

### 1.4 NeuroD1 treatment reduced seizure activity

We next investigated whether such neuroregenerative gene therapy might have any therapeutic potential in the TLE rats. We performed continuous video-electroencephalographic (video-EEG) recordings for 5 days, starting from 8 weeks after virus injection (Fig. 13). The untreated TLE rats or TLE rats injected with hGFAP: :GFP control virus displayed spontaneous EEG seizures with high-frequency and high-voltage synchronized poly-spikes (Fig. 13a), but significantly inhibited in NeuroD1-GFP group (Fig. 13b). EEG seizure activity was confirmed by simultaneous video recording showing rearing and falling of the TLE rats (Racine scale 4-5). Furthermore, EEG data were processed by Short-time Fourier transform to verify seizure activity (Fig. 13c), which was set as the average power in the testing time window being twice higher than the resting state. Power spectrum analysis revealed that the peak amplitude of the power of seizure bursts was around 5-12 Hz (Fig. 13d). Quantitative analysis of the spontaneous recurrent seizures (SRS) demonstrated that SRS was not observed in sham rats without pilocarpine injection (Fig. 13e, sham), but TLE rats showed significant numbers of SRS (Fig. 13e, TLE, 3.58 ± 4.28 seizures per day, n = 13 rats). After NeuroD1 treatment, there was a 90% reduction in the SRS (Fig. 13e; 0.37 ± 0.62 seizures per day, n = 14 rats; P < 0.005 compared with TLE, one-way ANOVA followed with Tukey post hoc test). Together, these results suggest that NeuroD1-converted GABAergic neurons may have therapeutic potential in inhibiting seizure activities of TLE rats.

### 1.5 NeuroD1 treatment rescued behavioral abnormalities

Because epileptic patients often suffer from cognitive impairment and behavioral abnormalities including psychiatric disorders and anxiety³⁶, we analyzed the effect of NeuroD1 treatment on some behavioral abnormalities in TLE rats. In the elevated plus maze test, while sham control rats rarely stayed long in the open arm, TLE rats spent much more time in the open arms than the sham rats (Fig 14a, sham: 7.4 ± 7.4%, 13 rats; TLE: 50.9 ± 36.7%, 14 rats, P < 0.0002). NeuroD1 treatment reversed this phenomenon (16.4 ± 16.9%, 12 rats; P < 0.003, compared with TLE rats, one-way ANOVA with Tukey post hoc test). We did not observe differences in the number of open arm entries among the three groups (Fig 14b, sham: 6.5 ± 4.8 times, 13 rats; TLE: 8.6 ± 10.0 times, 14 rats; TLE+NeuroD1: 8.3 ± 6.9 times, 12 rats; P = 0.75, one-way ANOVA). In open field test, TLE rats with or without NeuroD1 treatment spent more time in the center of the arena than the sham control rats (Fig 14c, center duration: sham: 7.36 ± 10.15 sec, 14 rats; TLE: 39.18 ± 40.27 sec, 15 rats; TLE+NeuroD1: 29.96 ± 27.01 sec, 14 rats; sham compared with TLE: P < 0.02, one-way ANOVA with Tukey post hoc test). The total distance travelled in the open field test showed a trend of increase in TLE and TLE+NeuroD1 groups (Fig 14d, sham: 2621 ± 1257 cm, 14 rats; TLE: 3771 ± 1873 cm, 15 rats; TLE+NeuroD1: 4208 ± 1963 cm; one-way ANOVA, P = 0.054), indicating a trend of hyperactivity in the TLE rats. In addition, rats with TLE have been shown to develop cognitive deficits³⁷. Therefore, we tested whether *in vivo* neuronal conversion might affect cognitive functions in TLE rats. In novel object recognition (NOR) test, TLE rats showed a significant decrease in the time spent exploring the novel object comparing to the sham rats (Fig 14e-f, sham: 72.4 ± 15.0%, 11 rats; TLE: 48.0 ± 17.1%, 16 rats, P < 0.002). This apparent deficit in NOR was reversed after NeuroD1 treatment (Fig 14e-f, 67.3 ± 14.2%, 11 rats, compared to TLE, P < 0.02, one-way ANOVA followed with Tukey post hoc test). In T-maze food retrieval test, TLE rats showed decreased correctness in comparison to sham control rats (Fig 14g-h, percentage of correctness: sham: 72.7 ± 20.5%, 11 rats; TLE: 54.7 ± 22.0%, 15 rats), which was significantly improved after NeuroD1-treatment (Fig 14g-h; NeuroD1 correctness: 75 ±17.3%, 12 rats; P < 0.04 compared to TLE rats, one-way ANOVA followed with Tukey post hoc test). In summary, these results suggest that NeuroD1 gene therapy treatment can ameliorate behavioral abnormalities following TLE.

### 1.6 Therapeutic intervention after long-term establishment of spontaneous recurrent seizures

The above experiments demonstrated that administration of AAV NeuroD1 at 1 week post pilocarpine-induced status epilepticus can effectively suppress epileptic seizures. Since many epileptic patients may not be able to access therapeutic treatment in a short time window after the onset of seizures, we further investigated whether it is possible to control seizure activity through neuroregenerative gene therapy after long-term establishment of spontaneous recurrent seizures (SRS). For this purpose, we performed video recordings at 3 weeks post pilocarpine administration for 5 consecutive days and then injected GFP control AAV or NeuroD1 AAV at 3 weeks post pilocarpine administration; at 7 weeks post viral injection, another 5 days of video were recorded, and immunostaining was performed at 8 weeks post viral infection to check cell conversion (Fig. 15a, schematic diagram illustrating the experimental design). In GFP group, 5 rats all showed SRS before viral injection, and two rats died due to severe epileptic seizures before 2^{nd} video recording. The remaining 3 rats all showed more severe epileptic seizures compared to before viral injection (Fig. 15b, top GFP group; quantified in Fig. 15c GFP bar graphs). In contrast, in NeuroD1 group, all 3 rats survived (Fig. 15d), and the seizure activity was significantly reduced after 7 weeks of NeuroD1 treatment (Fig. 15b, bottom ND1 group; quantified in Fig. 15c). At 8 weeks post viral injection and after 2^{nd} video recording, immunostaining confirmed that in the GFP control group, GFP alone infected cells remained astrocytic morphology and colocalized with GFAP (Fig. 15e; Fig. 16); whereas in the NeuroD1 group, NeuroD1-infected cells had become NeuN+ neurons with typical neuronal morphology (Fig. 15e; Fig. 16). These results suggest that NeuroD 1-based gene therapy treatment not only can have therapeutic effect on short-term seizure activity but also can have the potential to treat long-term seizure activity.

### 2. Methods

**Rats.** Male Sprague-Dawley (SD, 190-240g) were used and purchased from Guangdong Medical Laboratory Animal Center, China. Animals were housed one per cage, in stainless cages under a 12-h/12-h light/dark cycle with controlled temperature (20-26 °C), humidity (50 ± 20%) and ad libitum access to food and water. The Animal Experiment Protocol listed below has been reviewed and approved by Laboratory Animal Ethics Committee of Jinan University, China (IACUC no.20181211-07).

**The temporal lobe epilepsy model was established by intraperitoneal injection of pilocarpine.** After an acclimation period of 2-4 days, animals were injected with 350 mg/kg pilocarpine (purchased from Guangzhou Weijia Technology Co., Ltd.) (intraperitoneal injection, the pilocarpine was dissolved in ultrapure water, 150 mg/ml). The animals were injected with 1 mg/kg scopolamine (purchased from Sigma Co.) (the scopolamine was dissolved in ultrapure water, 1 mg/ml) half an hour in advance. Diazepam (purchased from the First Affiliated Hospital of Jinan University) (5 mg/ml) was used to terminate the acute seizure one hour after the first seizure. This experimental design allowed us to obtain TLE spontaneous recurrent seizure model with high efficiency.

**Adeno-associated virus (AAV) injection.** AAV was used to deliver transcription factor into the rat hippocampus with TLE. The AAV9 hGFAP::GFP and hGFAP::NeuroD1-P2A-GFP were used as viral vectors to express transcription factor NeuroD1 and green fluorescent protein (GFP) under control of the human GFAP promoter. AAV was injected stereotactically into both sides of the rat hippocampus (3 µl, 0.5-3 × 10⁹ GC/ml). Injection coordinates are as follows: anterior/posterior, -4.2 mm; medial/lateral, ±2.5 mm; and dorsal/ventral from skull, -3.2 mm.

**Chronic electroencephalographic (EEG) recordings.** TLE rats injected with either PBS, hGFAP::GFP or hGFAP::NeuroD1-GFP (AAV9) into both sides of the hippocampus for 1 week were implanted with electrodes for chronic EEG recordings. The animals were anesthetized with isoflurane (purchased from RWD Co,) (1-2.5%). Digital EEG activity was monitored daily for up to 5 days during 24-h sample recordings. A digital video camera was used to simultaneously monitor behavior during the EEG recordings. All recordings were carried out at least 3 days after surgery on rats freely moving in the cage. Data was collected by Spike2 8.0.

### Short-time Fourier transform (STFT) and continuous wavelet analysis.

Electroencephalographic data was calculated by OriginPro 9.1. The sampling rate was 250 Hz, and 15000 samples were input with a duration of 60 s. FFT Length = 256, Window Length = 256, Overlap = 128. Window type was Gaussian while the alpha value was 100.

**Behavioral analysis.** All behavioral tests were conducted during the light phase of the light/dark cycle. Rats injected for 9 weeks with either PBS, hGFAP::GFP or hGFAP::NeuroD1-GFP (AAV9) into both sides of the hippocampus were assessed with the open field test, the novel object recognition, the T maze and the elevated plus maze test. All behaviors were performed and analyzed by two investigators who were blinded to the treatment condition of the animals.

**Open field test (OFT).** The OFT was utilized to examine locomotor activity such as motor ability or anxious behavior. Rats were placed in a 100 × 100 × 50 cm black acrylic open field arena for 10 min under standard overhead lighting conditions. The behavior was recorded and analyzed by TopScan Lite software, including total distance, entries, velocity and latency to center and periphery. The size of the centre region is 60 × 60 cm.

**Novel object recognition.** A 50 cm × 50 cm × 50 cm open-top arena made of black acrylic was used for recognition testing. Square pyramidal and conical wooden blocks were used as objects with equivalent height, size, odor, color and texture. The two objects were placed in two corners diagonally away from the nearest wall at a distance of 12.5 cm to ensure the observation and exploration of rats. During training sessions, two familiar objects were placed into the open field and animals was allowed to explore for 10 min. During testing sessions, one of the familiar objects was replaced by a novel object and animals was allowed to explore for 5 min. Novel object preference was shown by a ratio of the amount of time spent exploring the novel object over the total time spent exploring both objects. The behavior was recorded and analyzed by TopScan Lite software.

**T-maze experiment.** The T-maze was made of black acrylic and had 3 closed arms of equal dimensions (50 × 10 × 25 cm). One of the arms was oriented perpendicularly to two opposed arms. Rats were placed in the vertical arm and face another two arms. During training sessions, rats were placed into the maze to adapt the environment in first day, then they were trained to remember the goal arm for three days. Each trial were completed in under 2 min. The test was conducted 24 hours after the training was completed. The training and testing were both repeated five times for each animal.

**Elevated plus maze experiment.** The elevated plus maze apparatus was comprised of two open arms (50 × 15 cm) and two closed arms (50 × 15 × 25 cm), elevated 65 cm above the floor level. Rats were placed in the central platform always facing the same open arm. Test duration was 5 min under standard overhead lighting conditions, rats that spent less time in the open arms were considered to have lower levels of risk-taking behavior. All data were collected using TopScan Lite software.

**Whole-cell patch-clamp recordings.** Hippocampal slices were prepared typically 6 weeks after virus injection and cut at 300 µm thick horizontal slices with a Leica vibratome (VT-1200S) in ice-cold cutting solution (containing 75 mM sucrose, 87 mM NaCl, 2.5 mM KCl, 0.5 mM CaCl2, 4 mM MgCl2, 24 mM NaHCO3, 1.25 mM NaH2PO4 and 25 mM glucose). Slices were incubated in NMDG-ACSF (containing 93 mM NMDG, 2.5 mM KCl, 1.2 mM NaH2PO4, 30 mM NaHCO3, 20 mM HEPES, 25 mM glucose, 5 mM sodium ascorbate, 2 mM Thiourea, 3 mM sodium pyruvate, 10 mM MgSO4•H2O, 0.5 mM CaCl2, pH 7.3 adjusted with HCl, 300-310 mOsm/L), continuously bubbled with 95% O2 and 5% CO2, first at 34 °C for 30 minutes, and then at room temperature. Whole-cell recordings were performed using Multiclamp 700B patch-clamp amplifier (Molecular Devices, Palo Alto, CA), and the slices were maintained in artificial cerebral spinal fluid (ACSF) containing 126 mM NaCl, 2.5 mM KCl, 1.25 mM NaH2PO4, 26 mM NaHCO3, 2 mM MgCl2, 2 mM CaCl2 and 10 mM glucose. The pH of bath solution was adjusted to 7.3 with NaOH, and osmolarity at 310-320 mOsm/L. Patch pipettes were pulled from borosilicate glass (~5-8 MΩ) and filled with a pipette solution consisting of 126 mM K-Gluconate, 4 mM KCl, 10 mM HEPES, 4 mM Mg₂ATP, 0.3 mM Na₂GTP, 10 mM Phospho-Creatnine (pH 7.3 adjusted with KOH, 290 mOsm/L). Data were collected using pClamp 10 software (Molecular Devices, Palo Alto, CA), sampled at 10 kHz and filtered at 3 kHz, analyzed with Clampfit 10.4.

**Immunohistochemistry staining.** AVV-injected rats were euthanized with 10% chloral hydrate and sequentially perfused with normal saline and 4% paraformaldehyde (PFA) in PBS. Brains were collected and postfixed with 4% PFA overnight at 4 °C. Postfixed brains were cryoprotected with 10-30% sucrose solution in PBS for 3 days. Staining was performed on free-floating 20 µm rat brain sections with the following antibodies: polyclonal anti-green fluorescent protein (GFP, chicken, 1:1000, Abcam, AB 13970), monoclonal anti-NeuN (rabbit, 1:1000, Abcam, ab177487), monoclonal anti-NeuroD1 (mouse, 1:500, Abcam, ab60704), polyclonal anti-Prox1 (rabbit, 1:500, Abcam, ab101851), monoclonal anti-GAD67 (mouse, 1:500, Sigma, MAB5406), monoclonal anti-parvalbumin (mouse, 1.5:1000, swant, 235), polyclonal anti-Somatostatin (rabbit, 1:2000, Peninsula Laboratories, T4103), polyclonal anti-Nitric Oxide Synthase (rabbit, 1:2000, sigma, N7280), monoclonal anti-calretinin (mouse, 1:500, swant, 6B3), monoclonal anti-calbindin-D-28K (mouse, 1:500, sigma, c9848), polyclonal anti-CCK-8 (rabbit, 1:500, sigma, c2581), DAPI (1:1000, Roche, 10236276001), Alexa Fluor 594 streptavidin for biocytin (Invitrogen, 1:500, s11227). Images were acquired with ZEN software (Zeiss) on AXIO Imager Z2 (Zeiss) and LSM880 confocal microscope (Zeiss).

**Timm staining.** 4 weeks after status epilepticus, brain tissues were sampled from the experimental animials, and for histological analysis of mossy fiber sprouting by Timm staining,. Rats were anesthetized and transcardially perfused with 1% sulfide solution. In brief, coronal serial section of 40µm were cut with freezing microtome and dried for more than 24hours in the air. The staining solution is composed of: 60ml 50% gum arabic, 10ml 2M sodium citrate buffer, 30ml 5.67% hydroquinone, and 0.5ml 17% silver nitrate solution. The sections were stained with Timm solution for 50-60min in darkness. After removal of the solution, the section were rinsed in tap water for 15min, dehydrated in graded alcohol to xylene, and mounted with DPX. The section were then observed and photographed with ZEN software (Zeiss) on AXIO Imager Z2 (Zeiss) microscope.

**Statistics.** Statistical analysis was performed with GraphPad Prism 6 and OriginPro 9.1. Statistical significance was tested with Student's two-tailed paired or unpaired *t* tests, two-tailed Mann-Whitney tests, and One-Way ANOVA test followed with Tukey post hoc test. All data are shown as mean ± s.d. The significance level was set to P < 0.05.

Other relevant experimental procedures can be found in CN104870634A, which is incorporated herein by reference.

### References

1. Toyoda, I., Bower, M.R., Leyva, F. & Buckmaster, P.S. Early activation of ventral hippocampus and subiculum during spontaneous seizures in a rat model of temporal lobe epilepsy. J Neuroscl 33, 11100-11115 (2013).
2. Kwan, P., et of. Definition of drug resistant epilepsy: consensus proposal by the ad hoc Task Force of the ILAE Commission on Therapeutic Strategies. Epilepsia 51, 1069-1077 (2010).
3. Stafstrom, C.E. Epilepsy comorbidities: how can animal models help? Adv Exp Med Biol 813, 273-281 (2014).
4. Shetty, A.K. & Upadhya, D. GABA-ergic cell therapy for epilepsy: Advances, limitations and challenges. Neurosci Biobehav Rev 62, 35-47 (2016).
5. Casalia, M.L., Howard, M.A. & Safaban, S.C. Persistent seizure control in epileptic mice transplanted with gamma-aminobutyric acid progenitors. Ann Neurol 82, 530-542, (2017).
6. Hunt, R.F., Girskis, K.M., Rubenstein, J.L, Alvarez-Buylla, A. & Baraban, S.C. GABA progenitors grafted into the adult epileptic brain control seizures and abnormal behavior. Nat Neurosci 16, 692-697 (2013).
7. Upadhya, D., et of. Human induced pluripotent stem cell-derived MGE cell grafting after status eplieptlcus attenuates chronic epilepsy and comorbidities via synaptic integration. Proc Natl Acad Sci U S A 116, 287-296 (2019).
8. Cunningham, M, et al. hPSC-derived maturing GABAergic interneurons ameliorate seizures and abnormal behavior in epileptic mice. Cell Stem Cell 15, 559-573, (2014).
9. Li, H. & Chen, G. In Vivo Reprogramming for CMS Repair: Regenerating Neurons from Endogenous Glial Cells. Neuron 91, 728-738 (2016).
10. Chen, G., et al. In Vivo Reprogramming for Brain and Spinal Cord Repair. eNeuro 2(2015).
11. Heinrich, C., SpagnoM, F.M. & Berninger, B. In vivo reprogramming for tissue repair. Nat Cell Biol 17, 204-211 (2015).
12. Chen, Y.C., et al. A NeuroD1 AAV-Based Gene Therapy for Functional Brain Repair after ischemic injury through in Vivo Astrocyte-to-Neuron Conversion. Mol Ther 28, 217-234 (2020).
13. Gascon, S., et al. identification and Successful Negotiation of a Metabolic Checkpoint In Direct Neuronal Reprogramming. Cell Stem Cell 18, 396-409 (2016).
14. Ge, L.J, et al. in vivo Neuroregeneration to Treat ischemic Stroke Through NeuroD1 AAV-Based Gene Therapy in Adult Non-human Primates. Front Cell Dev Biol 8, 5900008 (2020).
15. Grande, A., et al. Environmental impact on direct neuronal reprogramming in vivo In the adult brain. Nat Commun 4, 237.3 (2013).
16. Guo, Z., et al. In vivo direct reprogramming of reactive glial cells into functional neurons after brain injury and in an Alzheimer's disease model. Cell Stem Cell 14, 188-2.02 (2014).
17. Niu, W., et al. In vivo reprogramming of astrocytes to neuroblasts in the adult brain. Nat Cell Biol 15, 1164-1175 (2013).
18. Zhang, L., et al. Development of Neuroregenerative Gene Therapy to Reverse Glial Scar Tissue Back to Neuron-Enriched Tissue. Front Cell Neurosci 14, 594170 (2020).
19. Xiang, Z., et al. Lineage tracing of direct astrocyte-to-neuron conversion in the mouse cortex. Neural Regen Res 16, 750-756 (2021).
20. Wu, Z, et al. Gene therapy conversion of striatal astrocytes into GABAergic neurons in mouse models of Huntington's disease. Not Commun 11, 1105 (2020).
21. Liu, Y., et al. Ascl1 Converts Dorsal Midbrain Astrocytes into Functional Neurons in Vivo. J Neurosci 35, 9336-9355 (2015).
22. Heinrich, C., et al. Sox2-mediated conversion of NG2 glia into Induced neurons in the injured adult cerebral cortex. Stem Cell Reports 3, 1000-1014 (2014).
23. Torper, O., et al. In Vivo Reprogramming of Striatal NG2 Glia, into Functional Neurons that integrate into Local Host Circuitry. Cell Rep 12, 474-431 (2015).
24. Pereira, M., et al. Direct Reprogramming of Resident NG2 Glia into Neurons with Properties of Fast-Spiking Parvalbumin-Containing Interneurons. Stem Cell Reports 9, 742-751 (2017).
25. Hu, X., et al. Region-Restrict Astrocytes Exhibit Heterogeneous Susceptibility to Neuronal Reprogramming. Stem Cell Reports 12, 290-304 (2019).
26. Tsunemoto, R, et al. Diverse reprogramming codes for neuronal identity. Nature 557, 375-380 (2018).
27. Pesaresi, M., Bonilila-Pons, S.A. & Cosma, M.P. In vivo somatic cell reprogramming for tissue regeneration: the emerging role of the local microenvironment. Curr Opin Cell Biol 55, 119-128 (2018).
28. Chourhane, M., et al. Lineage Reprogramming of Astroglial Cellis from Different Origins into Distinct Neuronal Subtypes. Stem Cell Reports 9, 162-176 (2017).
29. Curia, G., Longa, D., Biagini, G., Jones, R.S. & Avoli, M. The pilocarpine model of temporal lobe epilepsy. J Neurosci Methods 172, 143-157 (2008).
30. Yu, J., Proddutur, A., Swietek, B., Elgartimal, F.S. & Santhakumar, V. Functional Reduction in Cannabinoid-Sensitive Heterotypic inhibition of Dentate Basket Cells in Epilepsy: Impact on Network Rhythms. Cereb Cortex (2015).
31. Puls, B., et al. Regeneration of dorsal spinal cord neurons after injury via in situ NeuroD1-mediated astrocyte-to-neuron conversion. BioRxiv October, 1-33 (2019).
32. Kuruba, R., Hattiangady, B. & Shetty, A.K. Hippocampal neurogenesis and neural stem cells in temporal lobe epilepsy. Epilepsy Behav 14 Suppl 1, 65-73 (2009).
33. Buckmaster, P.S., Abrams, E. & Wen, X. Seizure frequency correlates with loss of dentate gyrus GABAergic neurons in a mouse model of temporal lobe epilepsy. J Comp Neurol 525, 2592-2610 (2017).
34. Santellto, M., Toni, N. & Volterra, A. Astrocyte function from information processing to cognition and cognitive impairment. Nat Neurosci 22, 154-166 (2019).
35. Bardehle, S., et al. Live imaging of astrocyte responses to acute injury reveals selective juxtavascular proliferation. Nat Neurosci 16, 580-586 (2013).
36. Brooks-Kayal, A.R., et al. Issues related to symptomatic and disease-modifying treatments affecting cognitive and neuropsychiatric comorbidities of epilepsy. Epilepsia 54 Suppl 4, 44-60 (2.013).
37. Lopes, M.W., et al. Time course evaluation of behavioral impairments in the pilocarpine model of epilepsy. Epilepsy Behav 55, 92-100 (2016).
38. Farmer, W.T. & Murai, K. Resolving Astrocyte Heterogeneity in the CNS. Front Cell Neurosci 11, 300 (2017).
39. Zeisel, A., et al. Brain structure. Cell types in the mouse cortex and hippocampus revealed by single-cell RNA-seq. Science 347, 1138-1142 (2015).
40. Morel, L., et al. Molecular and Functional Properties of Regional Astrocytes in the Adult Brain. 1 Neurosci 37, 8706-8717 (2017).
41. Anderson, N.C., et al. Pluripotent stem cell-derived interneuron progenitors mature and restore memory deficits but do not suppress seizures In the epileptic mouse brain. Stem Cell Res 33, 83-94 (2018).
42. Garrets, E., Sanchez, S., Lajara, J., Montserrat, N. & Belmonte, J.C.I. Roadblocks in the Path of IPSC to the Clinic. Curr Transplant Rep 5, 14-18 (2018).
43. Kullmann, D.M., Schorge, S., Walker, M.C. & Wykes, R.C. Gene therapy in epilepsy-is it time for clinical trials? Not Rev Neurol 10, 300-304 (2014).
44. Snowball, A., et al. Epilepsy gene therapy using an engineered potassium channel. J Neuroscl (2019).
45. Katiel, D., Nicholson, E., Scharge, S., Walker, M..C. & Kullmann, D.M. Chemical-genetic attenuation of focal neocortical seizures. Not Common 5, 3.847 (2014).
46. Paz, J.T, et al. Closed-loop optogenetic control of thalamus as a tool for interrupting seizures after cortical, injury. Not Neurosol 16, 64-70 (2013).
47. Krook-Magnuson, E., Armstrong, C., Oijala, M. & Soltesz, I. On-demand optogenetic control of spontaneous seizures in temporal lobe epilepsy. Atat Commun 4, 1376 (2013).
48. Lieb, A., et al. Biochemical autoregulatory gene therapy for focal epilepsy. Not Med 24, 1324-1.329 (2018).
49. George Paxinos, C.W. The Rat Brain in Stereotaxic Coordinates. (1998).

### Equivalent Solutions

It should be understood that, while the present invention has been described in conjunction with specific embodiments thereof, the foregoing description is intended to illustrate rather than limit the scope of the present invention. The scope of the present invention is defined by the scope of the appended claims. Other aspects, equivalents, variations and advantages of the present invention are within the scope of the appended claims.

### SEQUENCE LISTING

| | |
|---|---|
| SEQ ID NO: 1 | Met Thr Lys Ser Tyr Ser Glu Ser Gly Leu Met Gly Glu Pro Gln Pro Gln Gly Pro Pro Ser Trp Thr Asp Glu Cys Leu Ser Ser Gln Asp Glu Glu His Glu Ala Asp Lys Lys Glu Asp Asp Leu Glu Ala Met Asn Ala Glu Glu Asp Ser Leu Arg Asn Gly Gly Glu Glu Glu Asp Glu Asp Glu Asp Leu Glu Glu Glu Glu Glu Glu Glu Glu Glu Asp Asp Asp Gln Lys Pro Lys Arg Arg Gly Pro Lys Lys Lys Lys Met Thr Lys Ala Arg Leu Glu Arg Phe Lys Leu Arg Arg Met Lys Ala Asn Ala Arg Glu Arg Asn Arg Met His Gly Leu Asn Ala Ala Leu Asp Asn Leu Arg Lys Val Val Pro Cys Tyr Ser Lys Thr Gln Lys Leu Ser Lys Ile Glu Thr Leu Arg Leu Ala Lys Asn Tyr Ile Trp Ala Leu Ser Glu Ile Leu Arg Ser Gly Lys Ser Pro Asp Leu Val Ser Phe Val Gln Thr Leu Cys Lys Gly Leu Ser Gln Pro Thr Thr Asn Leu Val Ala Gly Cys Leu Gln Leu Asn Pro Arg Thr Phe Leu Pro Glu Gln Asn Gln Asp Met Pro Pro His Leu Pro Thr Ala Ser Ala Ser Phe Pro Val His Pro Tyr Ser Tyr Gln Ser Pro Gly Leu Pro Ser Pro Pro Tyr Gly Thr Met Asp Ser Ser His Val Phe His Val Lys Pro Pro Pro His Ala Tyr Ser Ala Ala Leu Glu Pro Phe Phe Glu Ser Pro Leu Thr Asp Cys Thr Ser Pro Ser Phe Asp Gly Pro Leu Ser Pro Pro Leu Ser Ile Asn Gly Asn Phe Ser Phe Lys His Glu Pro Ser Ala Glu Phe Glu Lys Asn Tyr Ala Phe Thr Met His Tyr Pro Ala Ala Thr Leu Ala Gly Ala Gln Ser His Gly Ser Ile Phe Ser Gly Thr Ala Ala Pro Arg Cys Glu Ile Pro Ile Asp Asn Ile Met Ser Phe Asp Ser His Ser His His Glu Arg Val Met Ser Ala Gln Leu Asn Ala Ile Phe His Asp |
| Amino acid seuqnce of human NeuroD1 356 AA | |
| SEQ ID NO: 2 | atgaccaaat cgtacagcga gagtgggctg atgggcgagc ctcagcccca aggtcctcca 60 agctggacag acgagtgtct cagttctcag gacgaggagc acgaggcaga caagaaggag 120 gacgacctcg aagccatgaa cgcagaggag gactcactga ggaacggggg agaggaggag 180 gacgaagatg aggacctgga agaggaggaa gaagaggaag aggaggatga cgatcaaaag 240 cccaagagac gcggccccaa aaagaagaag atgactaagg ctcgcctgga gcgttttaaa 300 ttgagacgca tgaaggctaa cgcccgggag cggaaccgca tgcacggact gaacgcggcg 360 ctagacaacc tgcgcaaggt ggtgccttgc tattctaaga cgcagaagct gtccaaaatc 420 gagactctgc gcttggccaa gaactacatc tgggctctgt cggagatcct gcgctcaggc 480 aaaagcccag acctggtctc cttcgttcag acgctttgca agggcttatc ccaacccacc 540 accaacctgg ttgcgggctg cctgcaactc aatcctcgga cttttctgcc tgagcagaac 600 caggacatgc ccccccacct gccgacggcc agcgcttcct tccctgtaca cccctactcc 660 taccagtcgc ctgggctgcc cagtccgcct tacggtacca tggacagctc ccatgtcttc 720 cacgttaagc ctccgccgca cgcctacagc gcagcgctgg agcccttctt tgaaagccct 780 ctgactgatt gcaccagccc ttcctttgat ggacccctca gcccgccgct cagcatcaat 840 ggcaacttct ctttcaaaca cgaaccgtcc gccgagtttg agaaaaatta tgcctttacc 900 atgcactatc ctgcagcgac actggcaggg gcccaaagcc acggatcaat cttctcaggc 960 accgctgccc ctcgctgcga gatccccata gacaatatta tgtccttcga tagccattca 1020 catcatgagc gagtcatgag tgcccagctc aatgccatat ttcatgatta g 1071 |
| Nucleotide sequence of human NeuroD1 1071 nt | |
| SEQ ID NO: 3 | Met Thr Lys Ser Tyr Ser Glu Ser Gly Leu Met Gly Glu Pro Gln Pro Gln Gly Pro Pro Ser Trp Thr Asp Glu Cys Leu Ser Ser Gln Asp Glu Glu His Glu Ala Asp Lys Lys Glu Asp Glu Leu Glu Ala Met Asn Ala Glu Glu Asp Ser Leu Arg Asn Gly Gly Glu Glu Glu Glu Glu Asp Glu Asp Leu Glu Glu Glu Glu Glu Glu Glu Glu Glu Glu Glu Asp Gln Lys Pro Lys Arg Arg Gly Pro Lys Lys Lys Lys Met Thr Lys Ala Arg Leu Glu Arg Phe Lys Leu Arg Arg Met Lys Ala Asn Ala Arg Glu Arg Asn Arg Met His Gly Leu Asn Ala Ala Leu Asp Asn Leu Arg Lys Val Val Pro Cys Tyr Ser Lys Thr Gln Lys Leu Ser Lys Ile Glu Thr Leu Arg Leu Ala Lys Asn Tyr Ile Trp Ala Leu Ser Glu Ile Leu Arg Ser Gly Lys Ser Pro Asp Leu Val Ser Phe Val Gln Thr Leu Cys Lys Gly Leu Ser Gln Pro Thr Thr Asn Leu Val Ala Gly Cys Leu Gln Leu Asn Pro Arg Thr Phe Leu Pro Glu Gln Asn Pro Asp Met Pro Pro His Leu Pro Thr Ala Ser Ala Ser Phe Pro Val His Pro Tyr Ser Tyr Gln Ser Pro Gly Leu Pro Ser Pro Pro Tyr Gly Thr Met Asp Ser Ser His Val Phe His Val Lys Pro Pro Pro His Ala Tyr Ser Ala Ala Leu Glu Pro Phe |
| Amino acid sequence of mouse NeuroD1 357 AA | |
| | Phe Glu Ser Pro Leu Thr Asp Cys Thr Ser Pro Ser Phe Asp Gly Pro Leu Ser Pro Pro Leu Ser Ile Asn Gly Asn Phe Ser Phe Lys His Glu Pro Ser Ala Glu Phe Glu Lys Asn Tyr Ala Phe Thr Met His Tyr Pro Ala Ala Thr Leu Ala Gly Pro Gln Ser His Gly Ser Ile Phe Ser Ser Gly Ala Ala Ala Pro Arg Cys Glu Ile Pro Ile Asp Asn Ile Met Ser Phe Asp Ser His Ser His His Glu Arg Val Met Ser Ala Gln Leu Asn Ala Ile Phe His Asp |
| SEQ ID NO: 4 | atgaccaaat catacagcga gagcgggctg atgggcgagc ctcagcccca aggtccccca 60 agctggacag atgagtgtct cagttctcag gacgaggaac acgaggcaga caagaaagag 120 gacgagcttg aagccatgaa tgcagaggag gactctctga gaaacggggg agaggaggag 180 gaggaagatg aggatctaga ggaagaggag gaagaagaag aggaggagga ggatcaaaag 240 cccaagagac ggggtcccaa aaagaaaaag atgaccaagg cgcgcctaga acgttttaaa 300 ttaaggcgca tgaaggccaa cgcccgcgag cggaaccgca tgcacgggct gaacgcggcg 360 ctggacaacc tgcgcaaggt ggtaccttgc tactccaaga cccagaaact gtctaaaata 420 gagacactgc gcttggccaa gaactacatc tgggctctgt cagagatcct gcgctcaggc 480 aaaagccctg atctggtctc cttcgtacag acgctctgca aaggtttgtc ccagcccact 540 accaatttgg tcgccggctg cctgcagctc aaccctcgga ctttcttgcc tgagcagaac 600 ccggacatgc ccccgcatct gccaaccgcc agcgcttcct tcccggtgca tccctactcc 660 taccagtccc ctggactgcc cagcccgccc tacggcacca tggacagctc ccacgtcttc 720 cacgtcaagc cgccgccaca cgcctacagc gcagctctgg agcccttctt tgaaagcccc 780 ctaactgact gcaccagccc ttcctttgac ggacccctca gcccgccgct cagcatcaat 840 ggcaacttct ctttcaaaca cgaaccatcc gccgagtttg aaaaaaatta tgcctttacc 900 atgcactacc ctgcagcgac gctggcaggg ccccaaagcc acggatcaat cttctcttcc 960 ggtgccgctg cccctcgctg cgagatcccc atagacaaca ttatgtcttt cgatagccat 1020 tcgcatcatg agcgagtcat gagtgcccag cttaatgcca tctttcacga ttag 1074 |
| Nucleotide sequence of mouse NeuroD1 1074 nt | |

## Claims

1. Use of a nucleic acid encoding a NeuroD1 polypeptide in the manufacture of a medicament for the treatment of temporal lobe epilepsy (TLE) in a subject, wherein the treatment comprises administering the nucleic acid encoding the NeuroD1 polypeptide to glial cells in hippocampus region of the subject, so that the glial cells express the NeuroD1 polypeptide and are converted into GABAergic neurons.

2. The use according to claim 28, wherein the glial cells are located in CA1 region of the hippocampus region.

3. The use according to claim 28 or 29, wherein the glial cells are astrocytes, optionally, reactive astrocytes.

4. The use according to claim 28 or 29, wherein the glial cells are NG2 glial cells, oligodendrocytes, or microglial cells.

5. The use according to any one of claims 28 to 31, wherein the NeuroD1 polypeptide is a human NeuroD1 polypeptide.

6. The use according to any one of claims 28 to 32, wherein the nucleic acid encoding the NeuroD1 polypeptide is present in a viral vector, optionally, an adeno-associated viral vector.

7. The use according to any one of claims 28-33, wherein the nucleic acid encoding the NeuroD1 polypeptide is operably linked to a promoter sequence, preferably, the promoter is a tissue-specific promoter, more preferably, the promoter is a glial cell-specific promoter.

8. The use according to claim 34, wherein the promoter is an astrocyte-specific promoter, preferably, the promoter is a glial fibrillary acidic protein (GFAP) promoter, more preferably, the promoter is a human glial fibrillary acidic protein (hGFAP) promoter.

9. The use according to claim 34 or 35, wherein the expression level of the nucleic acid encoding the NeuroD1 polypeptide gradually decreases in converted GABAergic neurons until it is not expressed in fully mature neurons.

10. The use of any one of claims 28-36, wherein at least about 30%, 40%, 50%, 60%, 70%, 80%, 90%, 95% of the glial cells expressing the NeuroD1 polypeptide are converted into GABAergic neurons.

11. The use according to any one of claims 28-37, wherein the GABAergic neurons are GAD67 positive.

12. The use according to claim 38, wherein the GABAergic neurons are positive for one or more of the following proteins: parvalbumin (PV), neuronal nitric oxide synthase (nNOS), somatostatin (SST), calretinin (CR), calbindin (CB), or cholecystokinin (CCK).

13. Use of a nucleic acid encoding a NeuroD1 polypeptide in the manufacture of a medicament for converting glial cells in hippocampus region of a living mammalian brain into GABAergic neurons, wherein the conversion comprises causing the glial cells to express the NeuroD1 polypeptide and to be converted into GABAergic neurons.

14. The use according to claim 40, wherein the mammal is a human.

15. The use according to claim 40 or 41, wherein the glial cells are located in CA1 region of the hippocampus region.

16. The use according to any one of claims 40-42, wherein the glial cells are astrocytes, optionally, reactive astrocytes.

17. The use according to any one of claims 40-42, wherein the glial cells are NG2 glial cells, oligodendrocytes, or microglial cells.

18. The use according to any one of claims 40-44, wherein the NeuroD1 polypeptide is a human NeuroD1 polypeptide.

19. The use according to any one of claims 40-45, wherein the nucleic acid encoding the NeuroD1 polypeptide is present in a viral vector, optionally, in an adeno-associated viral vector.

20. The use according to any one of claims 40-46, wherein the nucleic acid encoding the NeuroD1 polypeptide is operably linked to a promoter sequence, preferably, the promoter is a tissue-specific promoter, more preferably, the promoter is a glial cell-specific promoter.

21. The use according to claim 47, wherein the promoter is an astrocyte-specific promoter, preferably, the promoter is a glial fibrillary acidic protein (GFAP) promoter, more preferably, the promoter is a human glial fibrillary acidic protein (hGFAP) promoter.

22. The use according to claim 47 or 48, wherein the expression level of the nucleic acid encoding the NeuroD1 polypeptide gradually decreases in converted GABAergic neurons until it is not expressed in fully mature neurons.

23. The use of any one of claims 40-49, wherein at least about 30%, 40%, 50%, 60%, 70%, 80%, 90%, 95% of the glial cells expressing the NeuroD1 polypeptide are converted into GABAergic neurons.

24. The use according to any one of claims 40-50, wherein the GABAergic neurons are GAD67 positive.

25. The use according to claim 51, wherein the GABAergic neurons are positive for one or more of the following proteins: parvalbumin (PV), neuronal nitric oxide synthase (nNOS), somatostatin (SST), calretinin (CR), calbindin (CB), or cholecystokinin (CCK).

26. Use of a NeuroD1 polypeptide in the manufacture of a medicament for treating temporal lobe epilepsy (TLE) in a subject, wherein the treatment comprises administering the NeuroD1 polypeptide into the interior of glial cells in hippocampus region of the subject, so that the glial cells are converted into GABAergic neurons.

27. Use of a NeuroD1 polypeptide in the manufacture of a medicament for converting glial cells in hippocampus region of a living mammalian brain into GABAergic neurons, wherein the conversion comprises administering the NeuroD1 polypeptide into the interior of the glial cells in the hippocampus region of the subject, so that the glial cells are converted into GABAergic neurons.
